# EUROPEAN PATENT APPLICATION

(11) **EP 4 844 665 A1**
(43) Date of publication of application: **25.11.2026**
(21) Application number: 26179758.3
(22) Date of filing: 20.05.2026
(51) Int. Cl.: G06Q 50/22, G16H 20/30, G16H 50/20

(54) **AI GREEN WELFARE CARD SYSTEM AND ITS OPERATION METHOD THAT PROVIDES PROCESSING AND SALES OF USER DATA, RECEIPT OF DONATIONS, PROVISION OF MARKETING MATERIALS, APP PORTALIZATION, PERSONALIZED MANAGEMENT AND DASHBOARD FUNCTIONS USING ARTIFICIAL INTELLIGENCE MODEL**

(30) Priority: 20.05.2025 KR 20250065693
(71) Applicant: RBIZ INC., Paju-si, Gyeonggi-do 10908 (KR)
(72) Inventor: PAE, Heecheol, 06701 Seocho-gu, Seoul (KR)
(74) Representative: ABG Intellectual Property Law, S.L.

(57) **Abstract**

A method of operating the AI green welfare card system based on user data collection and provision is disclosed. The method may include the steps of: transmitting raw data about a user by a user sensor to a server; obtaining, by the server, predicted data about the user by inputting the received raw data into the data prediction model when the raw data about the user is received from the user sensor; transmitting, by the server, integrated data including the raw data and the predicted data to an affiliated institution terminal; transmitting, by the server, an electronic voucher to a user terminal corresponding to the user sensor; and outputting the electronic voucher received from the server by the user terminal to provide the same to the user.

## Description

### TECHNICAL FIELD

The present disclosure relates to an artificial intelligence-based system, and more particularly, to an electronic device for implementing an AI green welfare card system that provides data obtained through collection and analysis of user data to an affiliated institution and provides information on an electronic voucher and a customized health management plan to the user, and an operation method thereof.

### BACKGROUND

As communication and network technologies have developed, various systems for managing personal information or user information in a server or the like have been developed.

User terminal devices such as smartphones, tablet PCs and the like have become widely popular, and a plurality of user terminals may individually perform communication connection with a server to transmit various information requests to the server, and provide visual or auditory information to users based on information received from the server.

Accordingly, the user can store information in a cloud server or the like instead of a personal storage space to overcome a limited storage space environment, and can easily manage and view information contained in various documents by storing the information in the form of digital data.

In addition, the viewing of such digital data has been diversified and specified in each field as user-friendly UI/UX technologies have been developed.

An artificial intelligence system is a computer system that implements human-level intelligence and is capable of self-learning and decision making, and whose recognition rate improves as it is used.

Artificial intelligence technology includes machine learning (deep learning) technology using an algorithm that classifies/learns features of input data by itself and element technologies that simulate functions of the human brain, such as cognition and determination, by utilizing a machine learning algorithm.

The elemental technologies may include at least one of a language understanding technology for recognizing human language/characters, a visual understanding technology for recognizing objects like humans do, an inference/prediction technology for determining and predicting information logically, a knowledge representation technology for processing experience information into knowledge data, and a motion control technology for controlling autonomous driving of vehicles or movements of robots.

Medical voucher programs contribute to increasing medical access for vulnerable populations but have limitations in funding and sustainability.

Existing systems fail to address privacy and legal compliance issues in data utilization, lack a revenue generation model, and lack features that encourage customer health management and sustained engagement.

### SUMMARY

### TECHNICAL PROBLEMS

The present disclosure has a purpose of providing an electronic device that inputs raw data collected through a user sensor into an artificial intelligence-based data prediction model to generate predicted data, provides the obtained data to an affiliated institution, and provides information on a personalized health management plan based thereon, and provides linked service for providing electronic vouchers.

The present disclosure can solve the above problems by integrating AI-based vital sign prediction, data sales, donation reception, marketing material provision, and app portalization, and adding a personalized management function and a dashboard function.

The purposes of the present disclosure are not limited to the above-mentioned purposes, and other objects and advantages of the present disclosure may be understood by the following description and will become more apparent from the embodiments of the present disclosure. In addition, it will be readily understood that the purposes and advantages of the present disclosure may be realized by means and combinations set forth in the claims.

### SOLUTIONS TO THE PROBLEMS

The electronic device for implementing the AI green welfare card system based on user data collection and provision according to this embodiment includes a communication interface for performing communication connection with an external device, a memory for storing user data and a data prediction model, and a processor connected to the communication interface and the memory to perform operations of collecting, processing, and providing user data, wherein the processor receives raw data about a user from a user sensor through the communication interface, inputs the received raw data into the data prediction model to obtain predicted data about the user, transmits integrated data including the raw data and the predicted data to an affiliated institution terminal through the communication interface, and transmits an electronic voucher to a user terminal corresponding to the user sensor through the communication interface.

The processor may receive raw data including at least one of a age, a gender, a location, a respiratory rate and a pulse rate of the users from the user sensor through the communication interface.

The processor may obtain predicted data on a body temperature and blood pressure of the user based on a value output by inputting the raw data into the data prediction model.

The processor may replace the user identifier corresponding to the integrated data with a predetermined identifier to obtain anonymized integrated data corresponding to the integrated data.

The processor may encrypt the integrated data in a predetermined manner to obtain encrypted integrated data corresponding to the integrated data.

When a target data request is received from the affiliated institution terminal through the communication interface, the processor may identify, among the integrated data, target data to be transmitted to the affiliated institution terminal based on the target data request, and transmit the target data to the affiliated institution terminal that has requested the target data through the communication interface.

The processor may transmit the integrated data to an affiliated institution terminal through the communication interface when receiving the payment information of the affiliated institutions through the communication interface.

The processor may transmit an electronic voucher corresponding to a high amount to the user terminal corresponding to the user sensor through the communication interface as the amount of raw data received from the user sensor through the communication interface increases, and transmit an electronic voucher corresponding to a low amount to the user terminal corresponding to the user sensor through the communication interface as the amount of raw data received from the user sensor through the communication interface decreases.

The processor may transmit an electronic voucher corresponding to a high amount through the communication interface to the user terminal corresponding to the user sensor as the accumulated usage amount of the voucher of the user increases and transmit an electronic voucher corresponding to a low amount through the communication interface to the user terminal corresponding to the user sensor as the accumulated usage amount of the voucher of the user decreases.

The processor may generate a customized health management plan for the user based on the raw data received from the user sensor through the communication interface and transmit the generated customized health management plan to the user terminal, wherein the customized health management plan includes at least one health management plan of food intake, exercise, and sleep.

The processor may generate dashboard information corresponding to the customized health management plan and transmit the dashboard information to the user terminal so that it is displayed.

The method of operating the AI green welfare card system based on user data collection and provision according to an embodiment of the present disclosure may include the steps of: transmitting raw data about a user by a user sensor to a server; obtaining, by the server, predicted data about the user by inputting the received raw data into the data prediction model when the raw data about the user is received from the user sensor; transmitting, by the server, integrated data including the raw data and the predicted data to an affiliated institution terminal; transmitting, by the server, an electronic voucher to a user terminal corresponding to the user sensor; and outputting the electronic voucher received from the server by the user terminal to provide the same to the user.

### ADVANTAGEOUS EFFECTS OF INVENTION

According to the present disclosure, sensor-based raw data collected from a user is processed through a data prediction model, thereby generating meaningful inference results rather than simple collection. The predicted data thus obtained is integrated with the raw data and transmitted to affiliated institutions such as hospitals and medical institutions so that the data can be usefully utilized.

In addition, since the automatic payment of an electronic voucher is made, a user may be provided with proper benefits by providing his/her biometric data.

The present invention can be utilized in various fields such as public welfare, a private partnership platform, and an AI-based service.

### BRIEF DESCRIPTION OF DRAWINGS

Aspects, features and advantages of certain embodiments of the present disclosure will become more apparent with the following description in conjunction with the accompanying drawings.
FIG. 1 is a diagram illustrating an example configuration of a system including an electronic device, a user sensor, a user terminal, and an affiliated institution terminal according to an embodiment of the present disclosure.
FIG. 2 is a block diagram illustrating the configuration of an electronic device according to an embodiment of the present disclosure.
FIG. 3 is a block diagram illustrating the configuration of a user sensor according to an embodiment of the present disclosure.
FIG. 4 is a block diagram illustrating the configuration of a user terminal according to an embodiment of the present disclosure.
FIG. 5 is a block diagram illustrating the configuration of an affiliated institution terminal according to an embodiment of the present disclosure.
FIG. 6 is a sequence diagram for describing representative operations of the system including an electronic device, a user sensor, a user terminal, and an affiliated institution terminal according to an embodiment of the present disclosure.
FIG. 7 is a block diagram illustrating user raw data sensed and obtained by the user sensor according to an embodiment of the present disclosure.
FIG. 8 is a block diagram illustrating an operation in which the electronic device inputs user raw data into a data prediction model to obtain predicted data such as body temperature and blood pressure according to an embodiment of the present disclosure.
FIG. 9 is a block diagram illustrating a process of anonymizing or encrypting integrated data including raw data and predicted data according to an embodiment of the present disclosure.
FIG. 10 is a diagram for describing operations of an electronic device, a user terminal and an affiliated institution terminal according to an embodiment of the present disclosure.
FIG. 11 is a diagram illustrating an example of a GUI for the health management plan and dashboard output by the user terminal through the display to provide the same to the user according to an embodiment of the present disclosure.
FIG. 12 is a diagram illustrating an example of a GUI for food intake information output by the user terminal and provided to the user through a display according to an embodiment of the present disclosure.
FIG. 13 is a diagram illustrating an example of a GUI for exercise information output by the user terminal and provided to the user through a display according to an embodiment of the present disclosure.
FIG. 14 is a diagram illustrating an example of a GUI for sleep information output by the user terminal and provided to the user through a display according to an embodiment of the present disclosure.
FIG. 15 is a block diagram illustrating the structure of a CRNN according to an embodiment of the present disclosure.
FIG. 16 is a flowchart illustrating a process example for implementing a multi-wallet system using a blockchain voucher network and explaining an actual payment example according to an embodiment of the present disclosure.
FIG. 17 is a flowchart illustrating a post-quantum cryptography process according to an embodiment of the present disclosure.
FIG. 18 is a flowchart for providing a health management plan based on dietary culture and climate data according to an embodiment of the present disclosure.
FIG. 19 is a flowchart illustrating an operation of the electronic device according to an embodiment of the present disclosure.

### MODE FOR CARRYING OUT THE INVENTION

Prior to describing the present disclosure in detail, a describing method for the present specification and drawings will be described

First of all, the terms used in this specification and claims have been selected considering functions in various embodiments of the present disclosure. However, these terms may vary depending on the intention of a person skilled in the art or legal or technical interpretation and appearance of new technology. In addition, some terms are arbitrary terms selected by an applicant. These terms may be interpreted as defined herein, and if there is no specific definition of a term, the terms may also be interpreted based on the overall content of the present specification and common technical knowledge in the relevant technical field.

In addition, the same reference numerals or signs in each of the drawings attached to this specification indicate parts or components that perform substantially the same function. For convenience of description and understanding, the same reference numerals or signs are used even in different embodiments. That is, although all elements having the same reference numerals in a plurality of drawings are illustrated, it does not mean that the plurality of drawings represent one embodiment.

In addition, in the specification and claims, terms including ordinal numbers such as "first", "second" may be used to distinguish between elements. Such ordinal numbers are used to distinguish identical or similar elements from each other, and the meaning of a term should not be limited by using such ordinal numbers. For example, an element combined with such ordinal numbers should not be restricted in use order or arrangement order by the number. If necessary, each ordinal number may be replaced with another one.

In the present specification, a singular form includes plural forms unless it is clear from context that the singular form is not to be interpreted as the plural form. In the present application, terms such as "include" or "comprise" are intended to indicate that features, numbers, steps, operations, elements, parts, or combinations thereof described in the specification exist, but do not preclude the existence or addition of one or more other features, numbers, steps, operations, elements, parts, or combinations thereof.

In the present disclosure, terms such as "module", "unit", and "part" refer to a component that performs at least one function or operation, which may be implemented by hardware or software, or a combination of hardware and software. In addition, unless each of the plurality of "modules", "units", and "parts" needs to be implemented with separate specific hardware, they may be integrated into at least one module or chip and implemented in at least one processor.

In addition, in the embodiments of the present disclosure, when a certain part is connected to another part, it includes not only direct connection but also indirect connection through other media. In addition, the meaning that a certain part comprises a specific component means that it may further include other components without excluding other components unless otherwise specified.

In the present disclosure, the expression "at least one of a, b or c" may refer to "a", "b", "c", "a and b", "a and c", "b and c", "all of a, b and c", or variations thereof.

The terms used in the present disclosure are selected from general terms currently widely used considering functions thereof, but may vary depending on intentions of those skilled in the art or judicial precedents, emergence of new technologies, etc. In addition, in certain cases, there are terms arbitrarily selected by an applicant, and the meanings thereof will be described in detail in corresponding description sections. Therefore, the terms used in the present disclosure should be defined based on the meanings of the terms and the overall content of the present disclosure, not merely as simple names of the terms.

The singular forms may include the plural unless the context clearly indicates otherwise. The terms used herein, including technical and scientific terms, may have the same meaning as commonly understood by those of ordinary skill in the art to which this disclosure pertains. In addition, although terms containing ordinal numbers such as 'first', 'second' are used to describe various components, these components should not be limited by such terms. These terms are only used to distinguish one component from another.

Throughout the specification, when an element is referred to as "including" a certain component, it should be understood that the element may further include other components without being limited thereto unless otherwise specified. In addition, terms such as "part" and "module" used in the specification refer to units processing at least one function or operation, which can be implemented by hardware, software, or a combination thereof.

Hereinafter, embodiments of the present disclosure will be described in detail with reference to the accompanying drawings so that those skilled in the art to which the present invention pertains can easily implement the same. However, it should be understood that the present disclosure is not limited to the specific embodiments set forth herein. In addition, parts unrelated to description are omitted from the drawings for clarity, and like numbers refer to like elements throughout the specification.

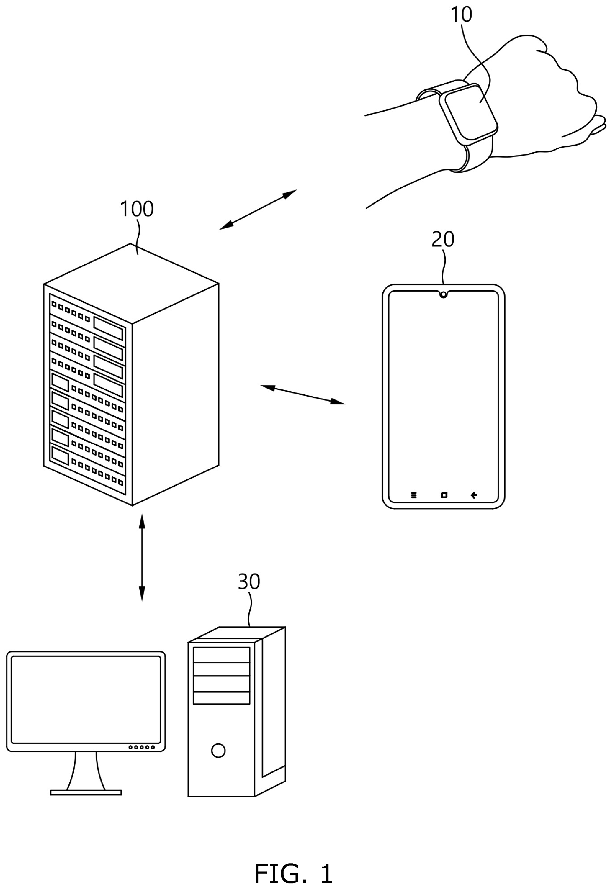

FIG. 1 is a diagram illustrating an example configuration of a system including an electronic device 100, a user sensor 10, a user terminal 20, and an affiliated institution terminal 30 according to an embodiment of the present disclosure.

Referring to FIG. 1, the server may be a device that performs various operations or functions, or may be a user terminal 20 or other devices.

When the electronic device 100 is a server, the electronic device 100 may be an FTP server (File Transfer Protocol Server), a web server, a database server, or a cloud server, but is not limited thereto. The server may perform one or more functions and operations, and may be implemented as a single device or distributed across multiple devices to implement each function and operation.

When the electronic device 100 is a user terminal 20, the electronic device 100 may be a smartphone, a tablet personal computer (tablet PC), a laptop personal computer (laptop PC), a desktop PC, a wearable device (e.g., a smart watch), a smart TV, a set-top box, a console, a dedicated virtual reality (VR) and augmented reality (AR) dedicated terminal (e.g., a head mounted device (HMD)), a smart home device, an industrial special terminal, or a vehicle terminal), but is not limited thereto. The user terminal 20 may perform one or more functions or operations, and each function or operation may be implemented by one device or distributed to various devices.

The electronic device 100 may perform communication with the user sensor 10, the user terminal 20, an external server, an external device, and/or an external terminal to transmit or receive various data, and perform operations of processing the obtained data.

At this time, the electronic device 100 may input the obtained data to an artificial intelligence model stored in a memory to obtain inference data or predicted data (PD) based on the output value.

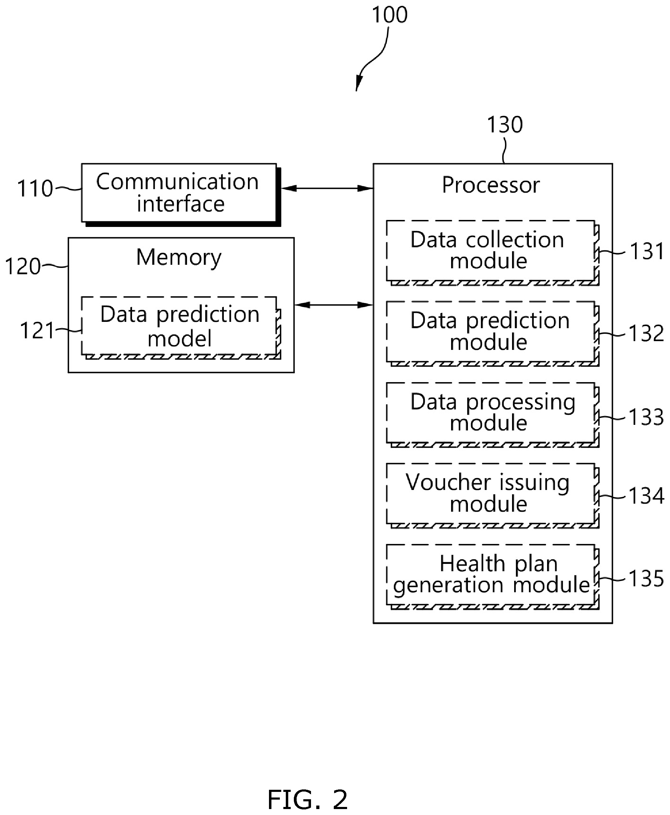

FIG. 2 is a block diagram illustrating the configuration of an electronic device 100 according to an embodiment of the present disclosure.

Referring to FIG. 2, the electronic device 100 may include a communication interface 110, a memory 120, and a processor 130. However, this is not limited thereto, and may further include other components or omit some components.

The communication interface 110 may include at least one of a wireless communication interface, a wired communication interface, and an input/output interface. The wireless communication interface may perform communication with various external devices using a wireless communication technology or a mobile communication technology. Examples of the wireless communication technology may include Bluetooth, Bluetooth Low Energy, CAN communication, Wi-Fi, Wi-Fi Direct, ultrawide band (UWB), ZigBee, Infrared Data Association (IrDA), or Near Field Communication (NFC), and examples of the mobile communication technology may include 3GPP, Wi-Max, Long Term Evolution (LTE), and 5G.

The wireless communication interface may be implemented using an antenna for transmitting electromagnetic waves to the outside or receiving electromagnetic waves from the outside, a communication chip, and a substrate.

The wired communication interface may perform communication with various devices based on a wired communication network. Here, the wired communication network may be implemented using a physical cable such as a pair cable, a coaxial cable, an optical fiber cable or an Ethernet cable.

One of the wireless communication interface and the wired communication interface may be omitted according to an embodiment. Therefore, the electronic device 100 may include only a wireless communication interface or only a wired communication interface. In addition, the electronic device 100 may include an integrated communication interface 110 that supports both a wireless connection through the wireless communication interface and a wired connection through the wired communication interface.

The electronic device 100 is not limited to including a single communication interface 110 performing communication connection in one manner, and may include a plurality of communication interfaces 110 performing communication connection in a plurality of manners.

The memory 120 temporarily or non-temporarily stores various programs and data, and transfers the stored information to the processor 130 according to a call of the processor 130. In addition, the memory 120 may store various information required for an operation, processing, or control operation of the processor 130 in an electronic format.

The memory 120 may include at least one of a main storage and an auxiliary storage. The main storage may be implemented using a semiconductor storage medium such as a ROM and/or a RAM. The ROM may include, for example, a conventional ROM, an EPROM, an EEPROM, or a MASK-ROM. The RAM may include, for example, a DRAM and/or a SRAM. The auxiliary storage may be implemented by using at least one storage medium capable of permanently or semi-permanently storing data, such as a flash memory device, a secure digital (SD) card, a solid state drive (SSD), a hard disc drive (HDD), a magnetic drum, an optical media such as a compact disc (CD), DVD, or a laser disc, a magnetic tape, an optical magnetic disk, and/or a floppy disk.

The memory 120 may store a data prediction model 121. Here, the data prediction model 121 may be an artificial intelligence model, a deep learning model, or a neural network model, but is not limited thereto.

The data prediction model 121 may be a model that outputs predicted data (PD) corresponding to the raw data RD when the raw data RD (biometric data, personal information, prescription information, disease information, allergy information, etc.) for the user is input, such as data on vital signs, e.g., body temperature D6 and blood pressure D7, but is not limited thereto.

The memory 120 may store an artificial intelligence model, output data obtained based on input data inputted to the artificial intelligence model, usage information obtained based on the output data, and reconstruction information for reconstructing the artificial intelligence model based on the usage information.

The artificial intelligence model may be constructed in consideration of an application field of the recognition model, a purpose of learning, or computer performance of the device. In addition, the artificial intelligence model may be, for example, a model based on a neural network.

The artificial intelligence model may be designed to simulate the structure of a human brain on a computer. The artificial intelligence model may include a plurality of network nodes having weights and simulating neurons of a neural network of a human being. The plurality of network nodes may form connection relationships with each other in order to simulate synaptic activity by which neurons exchange signals through synapses.

The artificial intelligence model may include a neural network model or a deep learning model developed from the neural network model. In the deep learning model, a plurality of network nodes may be located at different depths (or layers) D3 and exchange data according to a convolutional connection relationship. For example, models such as a deep neural network (DNN), a recurrent neural network (RNN), or a bidirectional recurrent deep neural network (BRDNN) may be used as an artificial intelligence model, but are not limited thereto.

The deep learning model may include a plurality of artificial neural network layers. The artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or one of the combinations of two or more of the above, but is not limited to the examples mentioned above. In addition to the hardware structure, the artificial intelligence model may additionally or alternatively include a software structure.

The deep learning model may include a learning algorithm. For example, the deep learning model may include supervised learning, unsupervised learning, semi-supervised learning, or reinforcement learning, but is not limited thereto. The artificial intelligence model may include a plurality of artificial neural network layers.

The processor 130 controls the overall operation of the electronic device 100. Specifically, the processor 130 is connected to components of the electronic device 100 including the memory 120 described above and may control the overall operation of the electronic device 100 by executing at least one instruction stored in the memory 120 as described above. In particular, the processor 130 may be implemented with a single processor or multiple processors.

The processor 130 may be implemented in various ways. For example, the one or more processors 130 may include at least one of a central processing unit (CPU), a graphics processing unit (GPU), an accelerated processing unit (APU), many integrated cores (MIC), a digital signal processor (DSP), a neural processing unit (NPU), a hardware accelerator, or a machine learning accelerator. The one or more processors 130 may control any combination of other components of the electronic device 100 and perform operations related to communication or data processing. The one or more processors 130 may execute one or more programs or instructions stored in the memory 120. For example, the one or more processors 130 may execute the one or more instructions stored in the memory 120 to perform a method according to an embodiment of the disclosure.

In embodiments of the present disclosure, the processor 130 may refer to a system on chip (SoC) in which one or more processors 130 and other electronic components are integrated, a single core processor, a multi-core processor, or a core included in a single core processor or a multi-core processor, where the core may be implemented as a CPU, GPU, APU, MIC, DSP, NPU, hardware accelerator, or machine learning accelerator, but embodiments of the present disclosure are not limited thereto.

The processor 130 may include a data collection module 131, a data prediction module 132, a data processing module 133, a voucher issuing module 134, and a health plan generation module 135.

Here, each module may be a configuration divided in hardware, but is not limited thereto and may share common hardware configurations while operating as different schemes, operations, or functions from the viewpoint of software control.

The data collection module 131 may be a module that performs an operation of obtaining integrated data (ID) including the raw data RD received through the communication interface 110 and predicted data (PD) output by inputting the raw data RD into the data prediction model 121.

The data prediction module 132 may be a module that performs an operation of obtaining predicted data (PD) based on the value output by inputting raw data RD to the data prediction model 121.

The data processing module 133 may be a module that performs an operation of anonymizing AID or encrypting EID the integrated data (ID), but is not limited thereto and may be a module that performs various operations of computing and processing data.

The voucher issuing module 134 may be a module for issuing, generating, and providing an electronic voucher EV to a user who provides raw data RD of the user. At this time, the electronic voucher EV may be a voucher that can be used instead of cash at affiliated institutions, health-related institutions, affiliated companies, and the like, but is not limited thereto.

The health plan generation module 135 may be a module for generating a customized health management plan HP corresponding to the raw data RD obtained through the user sensor 10. The health management plan HP may include information on food F intake, exercise E, sleep S, and the like, but is not limited thereto.

The user sensor 10 may be a wearable device that is disposed close to one area of the user's body or worn by the user on one area of the body such as a wristband or smart watch. However, it is not limited thereto and may be a device positioned at a location D3 within a predetermined range from an area of the user's body, or worn by the user to obtain biometric data based on sensing values obtained through sensors, and transmit the obtained biometric data (raw data RD) to the electronic device 100.

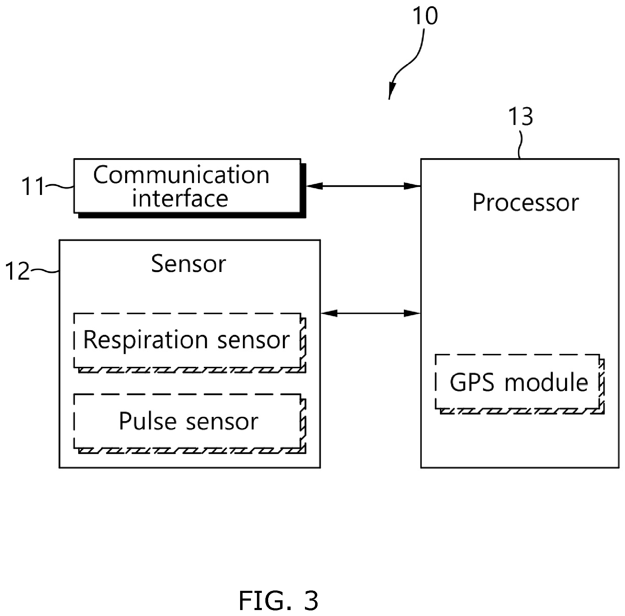

FIG. 3 is a block diagram illustrating the configuration of a user sensor 10 according to an embodiment of the present disclosure.

Referring to FIG. 3, the user sensor 10 may include a communication interface 11, a sensor 12, and a processor 13, but is not limited thereto and may further include other configurations or omit some configurations.

The general details of the communication interface 11 are the same as those of the communication interface described above in relation to FIG. 2.

The sensor 12 may detect an external environment state (e.g., a user state), and generate an electric signal or data value corresponding to the detected state.

According to an embodiment, the sensor 12 may be a biosensor (e.g., heart rate (pulse) sensor, blood pressure D7 sensor, brain wave sensor, respiration sensor, etc.), but is not limited thereto and may further include an acceleration sensor, angular velocity sensor, gyro sensor, proximity sensor, or the like.

The processor 13 may obtain information on the respiratory rate D4, pulse rate D5, and the like of users who wear the user sensor 10 through the sensor 12 and transmit the location D3 information of the user based on the GPS information to the electronic device 100 through the communication interface 11.

The user terminal 20 may be a terminal device corresponding to the user wearing the user sensor 10.

The user terminal 20 may be a smartphone, a tablet personal computer (tablet PC), a laptop personal computer (laptop PC), a desktop PC, a wearable device (e.g., a smart watch), a smart TV, a set-top box, a console, and a virtual reality (VR) and augmented reality (AR) dedicated terminal (e.g., a head mounted device (HMD)), a smart home device, an industrial special terminal, or a vehicle terminal, but is not limited thereto. The user terminal 20 may perform one or more functions or operations, and each function or operation may be implemented by one device or distributed to various devices.

The user terminal 20 may output the electronic voucher EV, customized health management plan HP, and dashboard DB information for monitoring and managing the health of a user received from the electronic device 100 to provide them as audiovisual contents.

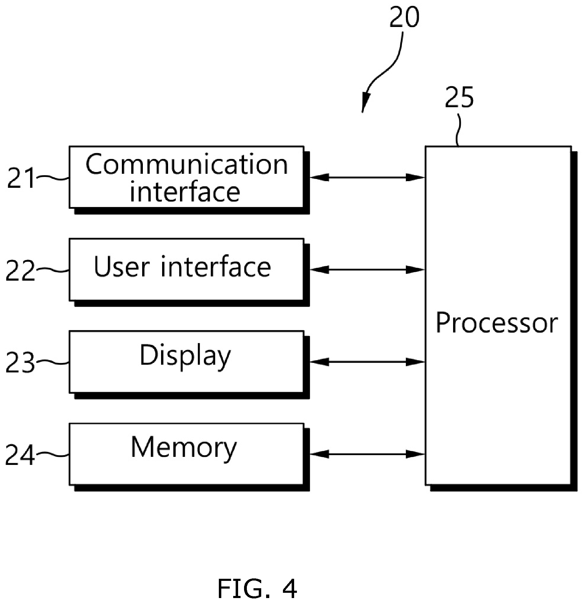

FIG. 4 is a block diagram illustrating the configuration of a user terminal 20 according to an embodiment of the present disclosure.

Referring to FIG. 4, the user terminal 20 may include a communication interface 21, a user interface 22, a display 23, a memory 24, and a processor 25, but is not limited thereto.

The processor 25 may perform communication connection with an external device, an external server, and a terminal through the communication interface 21 to transmit or receive various data, signals, etc.

The user interface 22 may include a button, a lever, a switch D3, a touch-type interface, a camera, a microphone, and a sensor (e.g., a gesture sensor, a motion sensor, a touch sensor, or a biometric sensor), and an input interface, but is not limited thereto.

The processor 25 may receive user input for data processing and data entry through the user interface 22.

Specifically, the processor 25 may receive a user input requesting an electronic voucher EV or a health management plan HP through the user interface 22, or a user input requesting raw data RD, predicted data (PD), etc., for the user.

The display 23 may include various types of display panels such as a liquid crystal display (LCD) panel, an organic light emitting diode (OLED) panel, an active matrix-organic light emitting diode (AM-OLED), a liquid crystal on silicon (LCoS), a quantum dot light-emitting diode (QLED), a digital light processing (DLP), a plasma display panel (PDP), an inorganic LED panel, and a micro LED panel, but is not limited thereto. Meanwhile, the display 23 may be configured to form a touch screen together with a touch panel or may be formed of a flexible panel.

The display 23 may be implemented in a square or rectangular shape of 2D, but is not limited thereto and may be implemented in various shapes such as a circle, polygon, 3D object, etc.

The display 23 may be disposed on one area of the surface of the user terminal 20, but is not limited thereto and may be implemented as a three-dimensional hologram projected onto a three-dimensional space or in the form of a projection projected onto a two-dimensional plane.

The processor 25 may output information on an electronic voucher EV, a health management plan HP, and a dashboard DB as visualized content through the display 23 to provide it to the user.

The affiliated institution terminal 30 may be an institution that is affiliated with the system for implementing the present embodiment, such as a hospital, medical center, pharmacy, pharmaceutical company, healthcare company, etc., but is not limited thereto.

The affiliated institution terminal 30 may be, for example, a smartphone, a tablet personal computer (tablet PC), a laptop personal computer (laptop PC), a desktop PC, a wearable device (e.g., a smart watch), a smart TV, a set-top box, a console, and a virtual reality (VR) and augmented reality (AR) dedicated terminal (e.g., a head mounted device (HMD)), a smart home device, an industrial special terminal, or a vehicle terminal, but is not limited thereto. The user terminal 20 may perform one or more functions or operations, and each function or operation may be implemented by one device or distributed to various devices.

The affiliated institution terminal 30 may receive the integrated data (ID) (personal information, biometric data, and the like) including the raw data RD and predicted data (PD) of the user from the electronic device 100.

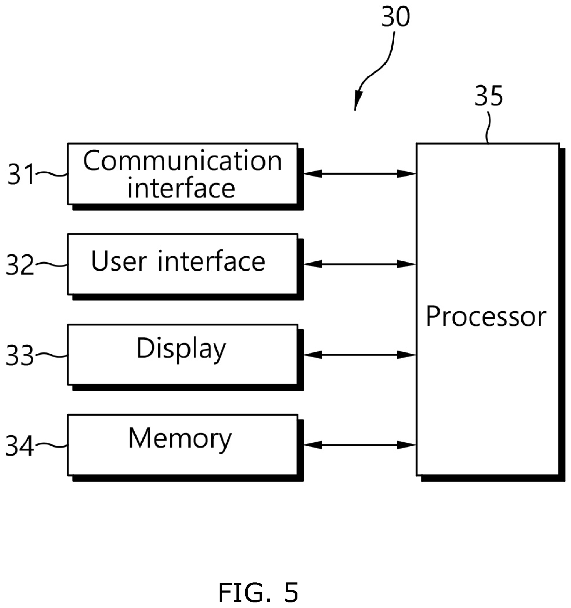

FIG. 5 is a block diagram illustrating the configuration of an affiliated institution terminal 30 according to an embodiment of the present disclosure.

Referring to FIG. 5, the affiliated institution terminal 30 may include a communication interface 31, a user interface 32, a display 33, a memory 34, and a processor 35, but the present disclosure is not limited thereto, and other components may be added or some of them may be omitted.

The processor 35 may perform communication with an external device or an external server through the communication interface 31 to receive raw data RD, predicted data (PD), and integrated data (ID) for a user.

In addition to the above, the processor 35 may receive or transmit various data, information and signals through the communication interface 31.

The processor 35 may receive a user input through the user interface 32 and perform a control operation based on the received user input.

The processor 35 may output and provide content in which the data is visualized through the display 33.

The memory 34 may store and database the raw data RD, predicted data (PD), etc., for a user.

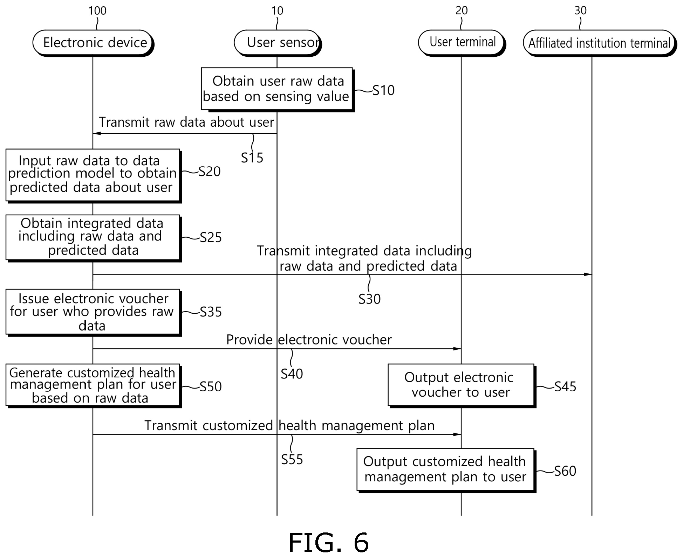

The operation method of the system including the above-described electronic device 100, user sensor 10, user terminal 20, and affiliated institution terminal 30 may be described with reference to FIG. 6.

FIG. 6 is a sequence diagram for describing representative operations of the system including an electronic device 100, a user sensor 10, the a terminal 20, and an affiliated institution terminal 30 according to an embodiment of the present disclosure.

Referring to FIG. 6, the user sensor 10 may obtain raw data RD of a user based on a sensing value of the sensor (S10).

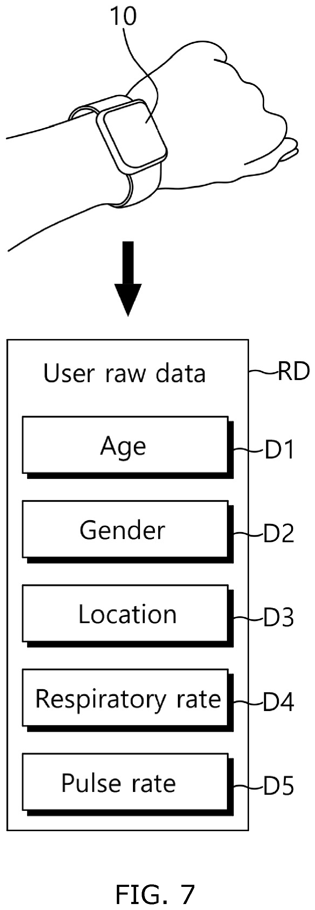

FIG. 7 is a block diagram illustrating user raw data RD sensed and obtained by the user sensor 10 according to an embodiment of the present disclosure.

Referring to FIG. 7, the raw data RD may be biometric data of a user, for example, a heart rate (a pulse rate D5), a respiratory rate D4, but is not limited thereto, and may include various biometric data such as step count, body temperature D6, blood pressure D7, and brain waves.

In addition, the user sensor 10 may obtain location D3 information of a place where the user sensor 10 is located D3 from the GPS server through the communication interface 11, and may obtain information on the user's age D1 and gender D2 based on information received through the communication interface 11 or user information input through the user interface.

The raw data RD may include data on an age D1, a gender D2, a location D3, a respiratory rate D4 and a pulse rate D5 of the users who wears the user sensor 10, but is not limited thereto, and may further include other data or information or may include some items omitted.

The user sensor 10 may transmit the obtained raw data RD to the electronic device 100 (S15).

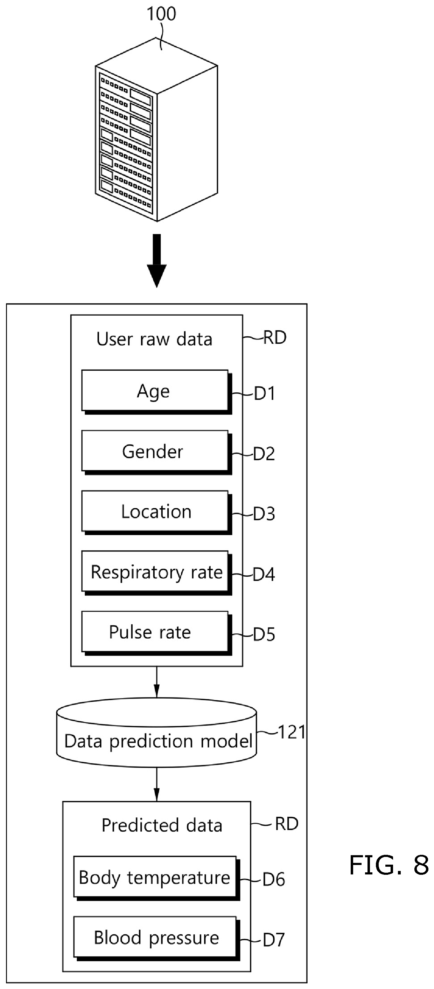

FIG. 8 is a block diagram illustrating an operation in which the electronic device 100 inputs user raw data RD into a data prediction model 121 to obtain predicted data (PD) such as body temperature D6 and blood pressure D7 according to an embodiment of the present disclosure.

Referring to FIG. 8, when the raw data RD is received from the user sensor 10, the electronic device 100 may input the raw data RD into a data prediction model 121 and obtain predicted data (PD) (S20). The electronic device 100 may obtain integrated data (ID) including the raw data RD and the predicted data (PD) (S25).

Here, the data prediction model 121 may be an artificial intelligence model, and the artificial intelligence model may include a neural network model or a deep learning model developed from the neural network model.

In the deep learning model, a plurality of network nodes may be located at different depths (or layers) D3 and exchange data according to a convolutional connection relationship. For example, models such as a deep neural network (DNN), a recurrent neural network (RNN), or a bidirectional recurrent deep neural network (BRDNN) may be used as an artificial intelligence model, but are not limited thereto.

The deep learning model may include a plurality of artificial neural network layers. The artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or one of the combinations of two or more of the above, but is not limited to the examples mentioned above. In addition to the hardware structure, the artificial intelligence model may additionally or alternatively include a software structure.

The deep learning model may include a learning algorithm. For example, the deep learning model may include supervised learning, unsupervised learning, semi-supervised learning, or reinforcement learning, but is not limited thereto. The artificial intelligence model may include a plurality of artificial neural network layers.

The electronic device 100 may obtain predicted data (PD) based on the vector value output from the data prediction model 121, and the predicted data (PD) may be data about vital signs including a body temperature D6 and blood pressure D7 of the user, but is not limited thereto.

At this time, the processor may perform a task of anonymizing AID or encrypting EID integrated data (ID) including raw data RD and predicted data (PD).

According to various embodiments of the present disclosure, the electronic device 100 may periodically evaluate prediction accuracy of an artificial intelligence model and perform a retraining operation when necessary.

For example of the AI green welfare care system, this system may analyze health data collected from a user device to provide a personalized health plan, and include a process of utilizing a machine learning model, periodically evaluating performance of the model, and retraining the model when necessary.

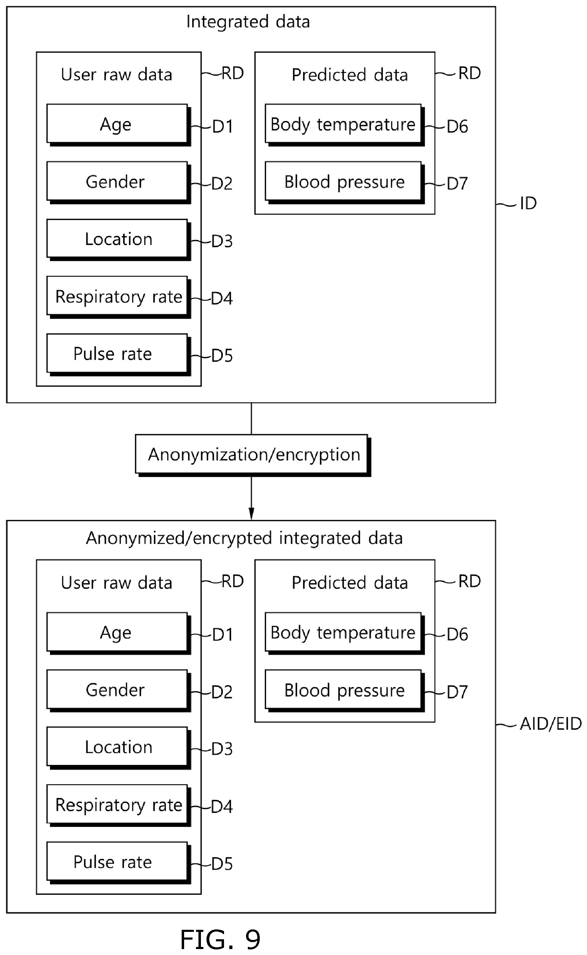

FIG. 9 is a block diagram illustrating a process of anonymizing AID or encrypting EID integrated data (ID) including raw data RD and predicted data (PD) according to an embodiment of the present disclosure.

Referring to FIG. 9, the electronic device 100 may replace the user identifier corresponding to the integrated data (ID) with a predetermined identifier to obtain an anonymized AID integrated data (ID) corresponding to the integrated data (ID).

The processing of anonymizing AID data may be referred to as pseudonymization, which is a method for replacing direct identifiers such as names and resident registration numbers with random codes or serial numbers so that individuals cannot be identified based on the information alone.

This method may be used to strengthen personal information protection while enabling data analysis or utilization by separately and securely storing information (such as a mapping table) that can identify the original data.

In addition to the above, various data anonymization methods may be used and anonymization is a technique for processing information that can identify an individual in data so that it is impossible to know who the specific person is. Representative anonymization techniques include pseudonymization, generalization, masking, data suppression, and data shuffling. Pseudonymization is a technique of replacing directly identifiable information such as names and resident registration numbers with arbitrary codes or IDs to minimize connection with original data. For example, this method replaces user 'Hong Gil Dong' with an arbitrary code such as 'User_1234'. Generalization is a method of grouping detailed information into larger categories to make it difficult to identify individuals, e.g., displaying age '27 years old' as '20s', and address 'Samsung-dong, Gangnam-gu, Seoul' as 'Seoul City'. Masking is a method of hiding directly identified information from data using symbols such as asterisks (*) or hash (#), for example, this method processes phone number '010-1234-5678' as '010-***-5678'. Data suppression is a method of completely removing fields that are easy to identify individuals, and data shuffling is a technique of randomly changing the order of data to reduce identification possibility.

The processor may encrypt EID the integrated data (ID) in a predetermined manner to obtain an encrypted EID integrated data (ID) corresponding to the integrated data (ID).

The technical method of encrypting EID personal information is a security technology that transforms data using a specific algorithm and key so that unauthorized persons cannot recognize the contents, and typically uses symmetric-key methods (such as AES) and asymmetric-key methods (such as RSA), and encrypted EID data cannot be decrypted without a decryption key, so personal information can be protected even if it is leaked during transmission or storage.

Encryption is a technique of converting original data into ciphertext through a specific algorithm and key so that the content cannot be recognized even if the data leaks to an unauthorized third party. The encryption may be classified into symmetric key encryption and asymmetric key encryption. In symmetric key encryption, the same key is used for both encryption and decryption, and representative examples thereof include Advanced Encryption Standard (AES). AES is a method of encrypting block unit data by using long keys such as 128 bits, 192 bits, or 256 bits through complex operations (substitution, bit mixing, etc.). In asymmetric key encryption, which is a type of cryptography in which encryption and decryption keys are different from each other, Rivest-Shamir-Adleman (RSA) is representative. RSA is a method of encrypting data with public key and decrypting it with private key based on mathematical problems (prime factorization). This method is suitable for securely transmitting data because data is encrypted with a publicly disclosed key. In addition, Data-at-Rest Encryption for encrypting data stored in storage and Data-in-Transit Encryption for encrypting data during transmission may be used together, for example, when applying an SSL/TLS protocol during data transmission, data is protected while being encrypted during network transmission.

The encryption EID scheme may be performed on the raw data of a user, predicted data, and various other data.

According to various embodiments of the present disclosure, the electronic device 100 may collect only a minimum amount of data required for achieving an objective in collecting data and establish a fine-grained consent system that allows the user to adjust a provision range. In this case, the electronic device 100 may limit essential data according to the principle of minimizing data.

The electronic device 100 may introduce role-based access control (RBAC) to separate data access rights into levels (e.g., anonymized data and named data), and the structure of the authority separation and roles may be defined as follows, including the following embodiments.

The administrator can access all data and change system settings, the physician can view/modify health data of his patients, the patient can only view his own health data, and the researcher can query anonymized data.

The electronic device 100 may designate a role when the user account is created by the administrator and change the above-described role through the user management interface, as necessary.

At this time, with respect to the access control mechanism, the electronic device 100 may perform an authentication operation by checking a role after verifying identity in a users table when a user logs in and check authority assigned to the role through a role_permissions table when making a work request.

For example, when the doctor requests to view health data of patient A, the electronic device 100 may receive login authentication information from the doctor and perform an authentication completion operation, and the role may be confirmed as "doctor".

The electronic device 100 may check the permission of "patient data viewing" for the role of "doctor" in the role_permissions table and allow the patient A to view his/her health data because the permission exists. At this time, if a patient attempts to view other patients' data, he/she may be blocked since the role of "patient" has only the permission of "viewing own data".

In the module implementation according to the above-described embodiment, a database may be implemented as follows: Users: (user_id, username, password_hash, role_id); Roles: (role_id, role_name); Permissions: (permission_id, permission_name); and role_permissions: (role_id, permission_id), and the implementation logic may be, at login, SELECT role_id FROM Users WHERE username = ? AND password_hash = ?, and at permission check, SELECT permission_id FROM role_permissions WHERE role_id = ? AND permission_id = ?, but is not limited thereto.

In this embodiment, the electronic device 100 may set a time-to-live (TTL) for each data type and implement an automatic suppression function after a period of time has elapsed. Specifically, the electronic device may include raw health data such as heart rate, blood pressure, sleep pattern, and the like collected from user devices and temporary logs including personal identifiable information (PII) or sensitive data generated for system operation or debugging purposes as objects to be suppressed.

The electronic device 100 may set a retention period of the raw health data to be 30 days from the collection date and a retention period of the temporary log to be 7 days from the generation date, and when the retention period expires, the data is automatically suppressed and may not be retained unless there are exceptions.

In addition, the electronic device 100 may include a reserved suppression scheme for performing data suppression as a suppression trigger, which includes checking a database every day to suppress data exceeding a retention period and additionally including an event-based suppression manner that immediately suppresses data when a specific event such as user account closure or a request of a user to delete data occurs. As a specific suppressing process, the electronic device 100 identifies data whose retention period has expired from a database, verifies data with a legal preservation flag set to exclude it from suppression, permanently suppresses identified data from the database and backup storage so that it is not recoverable, and records a date of suppression, a type of data, and a reason for suppression in audit logs to be preserved for at least one year.

In addition, the electronic device 100 may store aggregated data for statistical purposes from which personal identifiable information is removed as an exception and a special case of the automatic suppression process indefinitely, suspend suppression until legal hold is released with respect to data related to litigation or investigation. In addition, anonymized data for research purpose may be additionally retained upon explicit consent of the user, and data required to meet audit requirements according to regulations may be retained beyond standard retention period.

As another example, the electronic device 100 may be configured to support user-driven data suppression by allowing a user to request data suppression through a system UI or customer service channel and suppress the corresponding data within 24 hours after receiving the request. After completion of the suppression, it may transmit a completion notification to the user so that the user can clearly confirm the suppression completion state.

Finally, the electronic device 100 may perform a regular audit to check whether the suppression process is compliant with the regulations on a quarterly basis for compliance of the data suppression process and use an automated monitoring tool that transmits a warning to an administrator when the suppression fails.

The electronic device 100 may also continuously maintain and document the suppression policy and audit logs in preparation for review by a regulatory agency.

In this embodiment, the electronic device 100 may implement a 'right to be forgotten' in which the user can request data suppression at any time. Specifically, the user may make a data suppression request through a system user interface or customer support channel of the electronic device, and when receiving such a suppression request, the electronic device 100 may permanently suppress all data of the user from the database and backup storage within 30 days from the reception point so that it is impossible to recover. In addition, for faster response, the processor may perform immediate suppression on specific data upon receipt of the user's request, thereby immediately responding to the user's data protection request.

In another embodiment, when the electronic device 100 shares user data with a third-party affiliated institution, it may implement a procedure for obtaining explicit consent from the user. Specifically, before transmitting data to the affiliated institution, the electronic device 100 may clearly guide detailed information on data sharing such as purpose of data transmission, items of data to be shared and information about the receiving affiliated institution on a system user interface of the user, and transmit data only after receiving an explicit and clear expression of consent from the user. Accordingly, the electronic device 100 may include a step of 'confirming user consent prior to data transmission' in order to ensure transparency of the process of data sharing and self-determination right of users regarding their data.

In this embodiment, the electronic device 100 may implement a data validity verification algorithm to ensure reliability and accuracy of sensor data collected from user devices.

Specifically, the electronic device 100 may perform range verification for determining validity of a data value based on a predefined normal range after receiving sensor data and classify data exceeding the range as abnormal data. In addition, it may calculate a Z-score of data by applying a statistical technique and determine that the corresponding data is an outlier when the corresponding Z-score exceeds a preset threshold (e.g., 3 or more), and may determine whether data is an outlier using an interquartile range (IQR).

In addition, data that is separated from the center of a naturally formed group by more than a specific distance may be identified as an outlier using machine learning based k-means clustering, or data exhibiting abnormal patterns may be detected using anomaly detection algorithms such as isolation forest and one-class SVM. These methods for detecting outliers may be applied individually or in combination depending on the characteristics of the data and requirements of the system.

In another embodiment, the electronic device 100 may implement an algorithm for assigning a quality score to sensor data based on results of performing the outlier detection method as described above. For example, the electronic device may evaluate normal data that is not classified as an outlier by the outlier detection method with a high quality score and assign low quality scores to data determined to be outliers according to statistical methods or machine learning-based methods depending on the level of reliability reduction of the corresponding data.

The quality score may be calculated as a numerical scale (e.g., from 0 to 100), and data having the quality score equal to or less than a predetermined reference value may be regarded as unreliable data and excluded from an analysis process or transmitted for separate review. As such, the electronic device 100 can efficiently identify inaccurate or unreliable data through data quality evaluation and achieve overall data analysis and performance optimization of the system.

In an additional embodiment, the electronic device 100 may include an outlier detection and quality score calculation algorithm to perform a sensor data validity verification process and evaluate data quality. Accordingly, the electronic device 100 may minimize errors or inaccuracies that may occur in a process of evaluating and analyzing health status using sensor data, and provide optimized data-based services to users.

In this embodiment, the electronic device 100 may calculate a quality score to evaluate reliability and accuracy of collected data. The quality score is evaluated based on accuracy, completeness, and consistency and represented by a number between 0 and 100. More specifically, the electronic device may evaluate how accurately the data reflects an actual value according to a calibration state of a sensor or an environment (e.g., temperature or humidity), and assign a weight of 40% to an accuracy item.

In addition, the electronic device 100 may check whether collected data is continuously obtained without any omission at a predetermined period to evaluate an integrity item and assign a weight of 30% thereto. For example, heart rate data may receive a high score in the integrity item if heart rate data is collected at regular intervals without being continuously omitted.

In addition, the electronic device 100 may evaluate whether data maintains a consistent pattern in time or logic and assign a weight of 30% to the consistency item. For example, if heart rate is abnormally rapidly changed or sensor value is suddenly measured unrealistically, it may be evaluated as low consistency and assigned a low score. The final quality score is determined by comprehensively calculating three evaluation criteria according to each weight, and the weights of respective items may be adjusted depending on the purpose of the system and the importance of data.

The electronic device 100 may perform outlier detection and quality score calculation for the health data, detect outliers in the health data using a Z-score or an IQR method, and calculate a quality score based on accuracy, completeness, and consistency of the health data.

The electronic device 100 may transmit the anonymized AID or encrypted EID integrated data (ID) to the affiliated institution terminal 30 (S30). At this time, the electronic device 100 may transmit the integrated data (ID) to the affiliated institution terminal 30 through the communication interface when receiving payment information P of the partner through the communication interface.

The electronic device 100 may issue an electronic voucher EV for the user who provides raw data RD (S35), and transmit the issued electronic voucher EV to the user terminal 20 (S40).

The electronic device 100 may evaluate the amount and quality of collected biometric data to induce participation in providing biometric data by a user and provide an electronic voucher based on the evaluation result. For example, when a user continuously provides biometric data such as heart rate, blood pressure, or oxygen saturation through the user sensor 10, the electronic device 100 evaluates continuity and accuracy of the data and then immediately or periodically provides an electronic voucher EV that can be used at a health management app, online shopping mall, or affiliated health food store to a user who has provided high-quality biometric data for a specific period (e.g., weekly or monthly).

The user terminal 20 may output and provide the information on the electronic voucher EV received from the electronic device 100 to a user (S45).

That is, the user may issue and provide an AI green welfare card in the form of a voucher to the user who has provided raw data RD, and in this case, the voucher serves as an electronic gift certificate that can be used like cash at affiliated institutions, affiliated companies, healthcare providers, etc.

The electronic device 100 may identify the amount of raw data RD received from the user sensor 10 at a preset period.

At this time, the longer a period of time during which the user sensor 10 provides raw data RD to the electronic device 100 and the more items in the raw data RD provided, the greater the amount of raw data RD that can be identified by the electronic device 100.

The electronic device 100 may transmit an electronic voucher EV corresponding to a high amount to the user terminal 20 corresponding to the user sensor 10 as the amount of raw data RD received from the user sensor 10 increases and may transmit an electronic voucher EV corresponding to a low amount to the user terminal 20 corresponding to the user sensor 10 as the amount of raw data RD received from the user sensor 10 decreases.

In addition to the above, the electronic device 100 may transmit to the user terminal 20 an electronic voucher EV corresponding to a value obtained by multiplying the base amount by a first weight (first weight = AX, where A is a constant and X is the amount of raw data RD) having a larger value as the amount of raw data RD received from the user sensor 10 increases, and may transmit to the user terminal 20 an electronic voucher EV corresponding to a value obtained by multiplying the base amount by a first weight having a smaller value as the amount of raw data RD received from the user sensor 10 decreases.

In addition to the above, the electronic device 100 may receive payment history information using a voucher from an external device or the user terminal 20 and identify information on the amount of vouchers used for each user, the amount of voucher payments, that is, the accumulated amount of voucher use by users at predetermined intervals.

The electronic device 100 may calculate a ratio of the actual voucher accumulated use amount to the voucher amount provided to the user terminal 20 at every predetermined period. That is, this becomes a measure indicating how much the user has used the actually provided voucher.

At this time, if the above-described ratio is equal to or greater than a threshold value, the electronic device 100 may identify the A value as an A* value which is a larger value than the A value.

The electronic device 100 may transmit an electronic voucher EV corresponding to the amount obtained by multiplying a basic amount by a first weight having a value that increases as the amount of raw data RD received from the user sensor 10 increases (first weight = (A*)X, where A* is a constant and X is the amount of raw data RD), to the user terminal 20, and may transmit, to the user terminal 20, an electronic voucher EV corresponding to the amount obtained by multiplying the basic amount by a first weight having a smaller value as the amount of raw data RD received from the user sensor 10 decreases.

At this time, if the above-described ratio is less than a threshold value, the electronic device 100 may identify the A value as an A** value which is a smaller value than the A value.

The electronic device 100 may transmit an electronic voucher EV corresponding to the amount obtained by multiplying a basic amount by a first weight having a value that increases as the amount of raw data RD received from the user sensor 10 increases (first weight = (A**)X, where A** is a constant and X is the amount of raw data RD), to the user terminal 20, and may transmit, to the user terminal 20, an electronic voucher EV corresponding to the amount obtained by multiplying the basic amount by a first weight having a smaller value as the amount of raw data RD received from the user sensor 10 decreases.

The electronic device 100 may transmit the change information to the user terminal 20 so that it is displayed when the value A for calculating the first weight changes at a predetermined period.

Therefore, the user may be more likely to actively use a voucher he/she has received and provide raw data RD and use the voucher in order to receive an additional voucher allocated at a higher rate. That is, there is technical significance in promoting provision of raw data RD and use of vouchers.

The electronic device 100 may transmit an electronic voucher EV corresponding to a high amount to the user terminal 20 corresponding to the user sensor 10 as the accumulated use amount of the voucher of the user increases and transmit an electronic voucher EV corresponding to a low amount to the user terminal 20 corresponding to the user sensor 10 as the accumulated use amount of the voucher of the user decreases.

In addition to the above, the electronic device 100 may periodically evaluate performance of a machine learning model (or an artificial intelligence model) used for analyzing health data of the user. Specifically, the electronic device 100 may set a specific period (e.g., every week or month), calculate evaluation indexes such as accuracy, sensitivity, and specificity between prediction results and actual results of the model during the corresponding period, and determine whether these indexes satisfy preset performance criteria.

In another embodiment, when it is determined that the performance evaluation result of a machine learning model falls below a preset standard, the electronic device 100 may diagnose a cause for performance degradation and retrain the corresponding model using new data to improve the performance of the model.

For example, by adding the recently collected user health data and actual result data thereto to the training dataset to retrain an existing model, the model can more accurately reflect the current distribution of user data, thereby improving analysis accuracy.

As an additional embodiment, the electronic device 100 may automate a process of evaluating performance and retraining a machine learning model to perform automatic retraining of the model without user intervention when performance falls below a predetermined level. As such, this enables continuous optimization of the performance of the model to maintain reliability and accuracy in data analysis. The amount of data used for retraining the model, the period of time between evaluations of performance, and the performance criteria can be freely adjusted according to operating conditions of the system and characteristics of the data used by the user.

In this embodiment, the electronic device 100 may collect feedback from an external device or a user terminal to improve a machine learning model and provide personalized services through the improved machine learning model. Specifically, the user may provide feedback on various aspects such as accuracy or convenience of a service, and the electronic device 100 may update various artificial intelligence models, machine learning models, and deep learning models disclosed in this embodiment by using collected feedback data.

For example, the electronic device 100 may add an error case or an inaccurate prediction case derived through user feedback to a training dataset and re-evaluate performance of the model based on this, and retrain the model, thereby achieving personalized service provision and accuracy improvement reflecting the user's actual experience.

The electronic device 100 may perform semantic analysis based on the output value by inputting the text included in the feedback information to an artificial intelligence model, thereby identifying a meaning of the text included in the feedback information.

The electronic device 100 may identify a first text corresponding to the positive meaning and a second text corresponding to the negative meaning among texts included in the feedback information.

The first text may include words such as good, like, positive, best, and help, and the second text may include words such as so-so, negative, dislike, etc., but is not limited thereto.

When the first ratio of the size of the first text data in the total size of all text data included in the feedback information is equal to or greater than a first threshold value (e.g., 50%), the electronic device 100 may not update the artificial intelligence model.

When the second ratio of the size of the second text data in the total size of all text data included in the feedback information is equal to or greater than a second threshold value (e.g., 30%), the electronic device 100 may add an error case or an incorrect prediction case derived from the user's feedback to the training dataset and re-evaluate and retrain the performance of the model based on this. At this time, the sum of the first ratio and the second ratio may be less than 100%, but it is not limited thereto.

In addition, if the second ratio is equal to or greater than the second threshold value and the second ratio is larger than the first ratio, the electronic device 100 may add, to the training dataset, 'all error cases or incorrect prediction cases' derived through user feedback, re-evaluate the performance of the model based on this, and retrain the model.

However, if the second ratio is equal to or greater than the second threshold value and the second ratio is less than the first ratio, the electronic device 100 may add, to the training dataset, 'randomly selected half (or a threshold ratio less than 100% of the whole) from among all error cases or incorrect predicted cases' derived through user feedback, re-evaluate the performance of the model based on this, and retrain the model.

That is, according to the above-described embodiment, when a ratio of negative evaluation is high, all feedback information may be considered for updating a model and when a ratio of negative evaluation is lower than that, only some feedback information may be considered for updating a model so as not to excessively update a model, thereby achieving stability of the model.

In another embodiment, when a new data type is introduced to the system, the electronic device 100 may perform model version management and A/B testing for concurrently operating an existing model and a new model for a predetermined period of time, comparing performance of the two models, and selecting an optimal model. Specifically, the electronic device 100 may provide analysis results of the existing model to some users in a user group, and provide analysis results of the new model to other users in the user group, thereby evaluating a difference (e.g., accuracy, sensitivity, or user satisfaction) between the two models in real time. Based on the evaluation result, the most excellent model may be finally selected and applied to all users.

As an additional embodiment, the electronic device 100 may support an affiliated institution to access health data of a user in real time by providing a standardized API for strengthening cooperation with the affiliated institution. The API provided by the electronic device may be designed based on a REST architecture so that the partner can query health data of a specific user or request a personalized health management plan.

Specifically, the API may include a function of retrieving health data of a specific user through GET /users/{user_id}/health-data or requesting a personalized health plan through POST /users/{user_id}/health-plan.

In terms of security, the access to the APIs is made through an authentication procedure based on OAuth 2.0, and the affiliated institution may obtain an access token from the authentication server using a client ID and secret issued in advance.

In addition, all data transfers may be encrypted via the HTTPS protocol and sensitive data may be further encrypted via an AES-256 algorithm.

In this embodiment, the electronic device may perform a data providing procedure for sharing data with an affiliated institution in a safe and efficient manner. Specifically, the affiliated institution transmits a client ID and secret information previously issued to the authentication server to perform an authentication request, and the authentication server confirms validity of the request and then issues an access token to the affiliated institution. The affiliated institution includes the issued access token in a header of an HTTP request and transmits the request to an API endpoint of the electronic device. After processing the request from the affiliated institution, the electronic device returns user health data (e.g., heart rate, blood pressure, sleep time, etc.) in a JSON format as a response.

For example, when the affiliated institution requests health data of a user having a user ID "12345", it may perform a request in the following format.
GET /users/12345/health-data
Authorization: Bearer <access_token>

In this regard, the electronic device 100 may provide response data in a following JSON format.
"user_id": "12345", "health_data": "heart_rate": 72, "blood_pressure": "120/80", "sleep_hours": 7.5, "timestamp": "2023-10-01T12:00:00Z"

Through the data providing procedure, an affiliated institution can access health data of users managed by an electronic device in real time using a standardized API and perform secure and accurate data interconnection in a state where security is enhanced.

Errors that may occur in the process of an API request and response can be defined as standard HTTP error codes such as 401 Unauthorized, 403 Forbidden, 404 Not Found, and the like to enable efficient and clear data interconnection with a affiliated institution.

In this embodiment, the electronic device 100 may configure and provide user health data in a customized data package form according to the type of affiliated institution, thereby enabling each institution to efficiently and safely utilize data within a range that meets its purpose.

The electronic device 100 may be configured to identify the type of an affiliated institution according to a predefined classification scheme in a registration procedure for the affiliated institution and automatically apply a data provision policy corresponding to the identified type.

First of all, the affiliated institution type may be defined as follows. A hospital performs a role of diagnosing and establishing a treatment plan for a patient's health condition, so it needs access to real-time biometric data and history information of individual patients. An insurance company performs risk analysis such as premium calculation, risk group classification, and disease occurrence probability based on user's health data, so it requires indicators and pattern-centered information about health conditions. A research institute requires large-scale de-identified datasets that can be used for population-based statistical analysis or artificial intelligence training data, and individual identification information must be excluded. A fitness center aims to compose customized exercise programs or provide feedback based on user's exercise history and activity amount, so daily activity data and goal-based information are central.

Accordingly, the electronic device 100 may provide a data package according to each affiliated institution type as follows.

The hospital data package may include not only high-precision biometric data such as a user's heart rate, respiratory rate, blood pressure, body temperature, and sleep time but also clinical information such as medical history, information on drugs being taken, and recent medical examination history. As such, the hospital can receive all information necessary for accurate diagnosis and treatment path setting.

The insurance use data package may include lifestyle-based health risk assessment indicators such as whether the user has chronic diseases, smoking/drinking habits, exercise frequency, and eating habits, as well as anonymous statistical data such as health trends by age group or health distribution by region. This data can be directly used for risk analysis and premium adjustment.

The data package for the research institution is configured based on biometric signal data and health records accumulated in units of thousands or tens of thousands, and provided after removing all direct identifiers such as name, contact information, and resident registration number. For example, it may include time series data of heart rate, body temperature, and blood pressure, and demographic attributes such as age group, gender, and region of the corresponding user, and be usefully utilized to perform statistical analysis or develop disease prediction models.

The data package for the fitness center includes activity data such as an average number of steps per day, change in heart rate, exercise time, and type of exercise, and may provide additional information such as achievement rate compared to user's goal, exercise intensity prediction, and recommended exercise routine based on the data. Such data is utilized for automatic adjustment of training programs within the center or for user-customized feedback.

These data packages are mapped to pre-configured provision policies within the electronic device, and when an affiliated institution requests data through an API, the appropriate data package is automatically selected and transmitted based on the type of the institution. In addition, the data is designed to be provided only within the scope of the user's prior consent, and may include a procedure for obtaining additional consent from the user regarding the provision range if necessary. As such, the electronic device can maintain a balance between personal information protection and practical data utilization while efficiently providing precise information that meets the purpose of each affiliated institution.

In this embodiment, the electronic device 100 may configure a customized data package in which a data providing range is differently set according to a type of an affiliated institution and a purpose for using data. As such, an affiliated institution can efficiently use only information corresponding to its business purpose, and sensitive user information is not unnecessarily provided excessively. The data package is automatically configured based on a predefined provision standard, and when an affiliated institution requests data, it includes only appropriate data items depending on a type of the affiliated institution.

For example, the hospital data package includes high-resolution detailed health data collected from a user in a configuration to support accurate diagnosis and treatment. The hospital data package may include biometric signal data such as heart rate, blood pressure, respiratory rate, body temperature, sleep pattern, etc., which are collected in real time or at regular intervals, and clinical records such as past medical history, prescription history, diagnostic code, etc. These data can be used by healthcare professionals to monitor patient status in real time or evaluate drug response, etc.

The insurance use data package is configured to focus on quantitative determination such as premium calculation, disease risk analysis and subscriber risk assessment. The electronic device provides lifestyle-based data including health risk indices such as chronic disease diagnosis status, recent health checkup results, exercise frequency, and diet evaluation, and may also provide anonymized auxiliary data such as age-group health trends and region-based statistics with individual identification information removed. Accordingly, the insurance company can perform rate determination, risk classification, and customized product design according to the health level of individual contract holders.

The data package for the research institution is configured for group-based analysis purposes such as artificial intelligence model training, epidemiological investigation, and policy establishment. In this case, the electronic device provides a large amount of anonymized dataset, and core vital data such as heart rate, sleep pattern, blood pressure, and body temperature are provided without individual identification information, and demographic attributes such as gender, age range, region, and BMI range are merged and provided. The research institution may use it to perform health group distribution analysis, disease prevalence analysis, and lifestyle habit-health correlation analysis.

The data package for the fitness center may include real-time exercise data such as daily activity amount of a user, average exercise time, heart rate change, and calories burned in an optimized configuration for an exercise purpose, and also may include customized exercise suggestion information based on achievement ratio compared to a goal set by an individual and trend of exercise intensity change. The center may use this to configure member-customized training programs or operate an automated exercise routine recommendation system.

The electronic device 100 is configured to adjust the type of data, a range, and a sensitivity level according to requirements of each institution, and provide all data only within an agreement level range associated with prior user consent. In addition, it may include a policy-based access control function that can dynamically adjust a data providing scope based on an authentication level of an affiliated institution, a purpose for using data, and whether or not policies are followed in real time. As such, the electronic device 100 can realize a sophisticated data providing structure satisfying both data utilization efficiency and personal information protection.

In this embodiment, the electronic device 100 may be designed to provide data packages in a differentiated manner according to types and access authority levels of affiliated institution terminal. The affiliated institution accesses the electronic device through an API to request data, and the electronic device 100 limits a range of data that each institution can view based on an institution type (e.g., hospital, insurance company, research institute, fitness center), which is defined in advance, and a scope of consent for providing data obtained from the user. As such, it is possible to prevent misuse of data and safely distribute sensitive information.

For example, a hospital may access detailed health data of the corresponding user based on explicit consent of the patient. The hospital may query high-precision information such as heart rate, blood pressure, body temperature, sleep pattern, medical history, and medication history in real time or batch mode for diagnosis and treatment purposes, and perform personalized medical care based thereon.

Meanwhile, an insurance company may only view a health risk index (e.g., whether or not there is a chronic disease, exercise and diet score, etc.) and anonymized statistical data for the purpose of evaluating the general health condition of the user. In this case, the electronic device verifies whether the information requested by the insurance company does not exceed the user's consent scope, and only processes the request to respond with limited data through the API.

All data transmission is performed through a secure API channel (HTTPS) after an authentication procedure (e.g., OAuth 2.0), and the electronic device 100 may control access rights in real time by comprehensively determining the type of affiliated institution, the level of authentication, the purpose of access, etc. Such a data providing method can be designed to simultaneously achieve personal information protection and efficiency of data linkage between institutions.

The electronic device 100 differentiates the scope of data packages provided according to types of an affiliated institution and provides detailed health data when the affiliated institution is a hospital or provides a health risk index and statistical data when the affiliated institution is an insurance company.

In this embodiment, the electronic device 100 may include an affiliated institution evaluation system that periodically evaluates data utilization methods and qualities of affiliated institutions and provides differentiated incentives based on the results.

The electronic device 100 may analyze how the affiliated institution utilizes the received health data for what purpose and in what manner, and calculate a reliability score of each institution by comprehensively considering utilization frequency of the data, suitability, security compliance, user feedback, etc.

For example, in the case of a hospital, if collected health data is accurately utilized for diagnosis and treatment purposes, and high satisfaction is received from users, reliability scores may be increased. Meanwhile, when an insurance company makes requests that are beyond the purpose of utilizing data or repeatedly performs excessive requests, it may receive low evaluation.

The electronic device 100 may adjust the data providing range based on the evaluation result or provide an incentive in a manner of granting priority to data provision or access rights for additional data packages based on quantified indicators.

In addition, the electronic device 100 transparently manages evaluation history and incentive records, and may adjust whether to maintain a long-term partnership or data provision contract terms based on the evaluation history. As such, the system can induce responsible use of data, stably maintain user personal information protection levels, and strengthen cooperation with reliable institutions.

In this embodiment, the electronic device 100 may include various motivation schemes to induce user participation and improve continuous use of the system. To induce long-term provision of biometric data and participation in health management activities by a user, the electronic device may integrally operate an interactive function based on reward, social communication structure, premium service policy based on usage period, etc.

First of all, the electronic device 100 may connect a user's daily participation to an interesting element through gamification elements. For example, when the user inputs biometric data every day or performs exercise, virtual rewards such as 'continuous participation badge', 'health goal achievement level', and 'weekly challenge completion score' may be provided, and the electronic device may provide actual electronic vouchers or additional content use rights in different amounts according to accumulated points or achievement levels. This contributes to improving data quality and continuously inducing voluntary participation by systematizing reward based on user behavior.

In addition, the electronic device 100 may provide a social function and community structure to activate interaction between users. A user may share his or her health goal in a community, participate in a 'group challenge' with users having the same goal, and naturally enhance activities such as inputting health data, performing exercise, and managing sleep through this process. The electronic device may visualize the result of the challenge in real time, and provide positive motivation among users through a group ranking, an encouragement message, and feedback functions. Such a participation structure based on social networking converts isolated health management into a communal experience, thereby improving sustainability and immersion.

In addition, the electronic device 100 may operate a long-term user loyalty program to provide premium services to users who continuously participate in the system for a certain period of time or longer. For example, users who have participated for three months or more consecutively may be given a VIP level and benefits such as an increased voucher reward unit price for data provision, improved accuracy of personal health reports, and 1:1 coaching service by professional medical staff may be provided differentially. The use period is automatically accumulated, and the electronic device may update the benefits according to each grade on a regular basis and notify the user thereof, thereby reducing the service dropout rate and inducing long-term participation.

As described above, the electronic device 100 may convert user participation from a short-term event-based type to a long-term and voluntary data provision basis by integrally operating the reward structure, social connectivity, and user loyalty strategy. As such, this enables simultaneous securing of data quality, user satisfaction, and platform sustainability of the health management system.

In this embodiment, the electronic device 100 may comprehensively reflect various personal characteristics such as genetic information, disease history, occupation and environmental factors, psychological state, etc., in order to analyze a user's health status more precisely and provide customized health management services. Such configuration serves as a basis for implementing a multi-dimensional and individualized health management system beyond simple analysis based on biometric data.

First of all, the electronic device 100 may analyze a disease group to which the user is genetically vulnerable, metabolic characteristics, and drug response information based on genetic test results provided or linked by the user. For example, according to caffeine metabolism genes or blood sugar sensitivity genes, different diet plans may be recommended, or exercise types may be adjusted as cardio exercise or strength training based on genetic information. In this process, the electronic device may consider that genetic information is sensitive information, thereby enhancing personal information protection by including high-strength encryption, an anonymization algorithm, a user consent-based access control function, etc.

In another embodiment, the electronic device 100 may provide a health management program specialized for chronic disease patients. For example, in the case of a diabetic patient, blood sugar change patterns and postprandial responses can be monitored in real time, and blood sugar prediction information before and after meal times can be visually provided, or a warning system for blood sugar control risks during exercise may be included. For a patient with hypertension, by providing a management program combined with blood pressure change trend analysis, salt intake monitoring, and a medication time reminder function, it is possible to implement customized health care support according to diseases.

As another example, the electronic device 100 may differentiate a health management plan by considering a user's occupation and life environment factors. For example, an office worker who works for a long time sitting down may be recommended to stretch at regular intervals during the day and strengthen his or her back muscles, and a user working night shifts may receive guidance on adjusting sleep hours and nutritional supplements for optimizing biological rhythms. In addition, in the case of users working in high-temperature/low-temperature environments, it may suggest plans including dehydration risk warnings, body temperature maintenance strategies, etc., based on external temperature and humidity data.

In addition, the electronic device 100 may integrate a psychological well-being element into a health management plan to function in a direction of improving an overall quality of life of the user. For example, based on data such as a change in heart rate or sleep quality and reduced activity amount of the user, stress level may be predicted, and when it is determined that the user has high stress, intervention for mental health improvement, such as recommending meditation content, training deep breathing before sleeping, and suggesting a digital detox schedule, may be performed. In addition, if the user responds to a questionnaire related to stress or records his/her psychological state through a diary input function, the electronic device 100 may provide more precise emotion analysis and customized psychological management service based thereon.

As described above, the electronic device 100 may provide a user with a multi-layered customized health management plan that considers genes, disease states, work environments, and psychological factors in an integrated manner, thereby enabling not only physical health but also mental stability and continuous improvement of life. This configuration has the potential to be extended into a premium health management platform or digital therapeutics-linked model in the future.

In this embodiment, the electronic device 100 includes a structure that monitors biometric data of the user in real time and performs an emergency response procedure immediately when an abnormal state is detected. As such, it can function as not only a simple health management system but also an emergency support platform capable of rapid response to an emergency situation.

First of all, the electronic device 100 may automatically recognize through an abnormal biometric data detection function that a biometric signal exceeds a predetermined threshold or shows a rapid change. For example, if heart rate is maintained at 30 bpm or less or 150 bpm or more, blood pressure rises or falls rapidly, or oxygen saturation drops to 85% or less, the electronic device 100 determines this as an abnormal state and immediately generates an emergency notification for subsequent action. The notification is classified into a high-risk level according to the internal logic of the system and transmitted outside without delay.

In another embodiment, the electronic device 100 may include a function of automatically transmitting location information of the user along with an emergency notification. For example, when an emergency notification is generated, the user equipment or wearable device uses GPS-based coordinate information collected to transmit current location in real time together with a notification message to a guardian, family member, or pre-registered emergency contact. In addition, the electronic device 100 may also transmit location information and basic health status summary data to a registered affiliated medical institution or regional emergency medical center upon occurrence of an emergency situation. As such, this enables actual rescue activities or subsequent actions to be performed faster and more accurately.

In addition, the electronic device 100 may provide a data linkage structure with an affiliated medical institution so that when receiving an emergency notification, the corresponding medical institution can immediately access basic health information of the user, allergy history, currently taken medication, recent biometric data trends, and the like. This function enables the medical institution to secure background information on the user before arriving at the scene or starting treatment, thereby performing safer and more accurate emergency response. The linkage is directed to a pre-affiliated medical institution, and access thereto is allowed in a limited manner through an API authentication procedure based on user consent.

Through such a series of structures, the electronic device 100 may automate an emergency response flow including abnormal state detection → emergency notification generation → location and status information transmission → medical institution linkage, and operate as a preventive healthcare system that protects user lives and reduces the risk of severe illness. This function may be utilized as a core function in smart healthcare services targeting high-risk users (e.g., elderly people, chronic patients, or lonely seniors) .

In this embodiment, the electronic device 100 may include a data access function that is linked to enable an affiliated medical institution to immediately check health data of the user when an emergency situation occurs for the user. This configuration is one of core safety functions of the AI Green Welfare Card system and can improve speed and accuracy of emergency response by implementing real-time emergency detection, automatic notification generation, secure data transmission, and immediate use by a medical institution in one integrated flow.

First of all, the electronic device 100 monitors a user's biometric data in real time and when an abnormal sign such as a sudden increase in heart rate, a decrease in blood pressure, or a drop in oxygen saturation is detected, it determines that this is an emergency situation and immediately transmits an automatic notification to an affiliated medical institution. This notification includes a temporary access link that allows access to the corresponding user data along with key summary information of the emergency state.

After receiving the emergency notification, a medical institution is securely connected to the system through an OAuth 2.0 authentication procedure that has been previously established with the system, and the system issues a one-time access token only for the corresponding medical institution when an emergency situation occurs. The token is valid only for specific user data and is configured to be usable for a limited time of about 30 minutes or less in general. By transmitting an API request including the token to the system, the medical institution can safely view health information on the corresponding user.

After verifying that the request is valid, the system returns the minimum core health data required for emergency treatment in a JSON format such as recent vital signs (heart rate, blood pressure, body temperature, etc.), medical history, currently taken medication, allergy information, and the like. This data can be directly linked to an emergency room system or medical consultation terminal of a medical institution and immediately used for on-site information analysis before rescue operation or immediate diagnosis upon arrival.

For security purposes, all response data is encrypted with HTTPS-based communication and in particular sensitive data fields are further encrypted with the AES-256 algorithm before transmission. In addition, all accessed data is logged into audit logs so that the user can later check through the system when, by whom, and for what purpose and in what manner the user's own data has been accessed. This feature effectively balances privacy protection and emergency response by satisfying single use, time limit and record transparency requirements altogether.

As a result, the electronic device 100 may evolve from a simple health monitoring system to a real-time health-emergency platform that is organically linked with an emergency medical system and effectively contributes to protecting high-risk users and strengthening cooperation with local community healthcare networks.

In this embodiment, the electronic device 100 may include an emergency notification and data interconnection structure that enables an affiliated medical institution to immediately and securely view health data of a user when an emergency situation occurs for the user. This function is designed to detect an emergency situation based on real-time monitoring and support rapid judgment and treatment by a medical institution.

The electronic device 100 continuously monitors the biometric data of a user such as heart rate, blood pressure, and oxygen saturation level, and determines that an emergency situation occurs when the predefined threshold is exceeded or there is a rapid change in the biometric data. When this situation is detected, the system automatically transmits an emergency notification to the registered affiliated medical institution. The notification includes summary information on the state of the user, and a one-time token for accessing the data of the corresponding user and an API access link.

The affiliated medical institution completes a pre-authentication procedure based on the OAuth 2.0 protocol to be connected with the system and is given a unique client ID and secret key. When an emergency occurs, the system issues a temporary access token that applies single use and time limit for the corresponding medical institution, which is activated only within a validity period of generally 30 minutes. The medical institution may request real-time health data of the corresponding user through an API request including the token.

The electronic device 100 verifies the validity of the token and the appropriateness of the request, and then securely returns minimum essential information such as recent biometric data, medical history, medication history, allergy information, etc., of the user to the medical institution in a JSON format. The corresponding information is automatically linked with an emergency system of the medical institution so that it can be immediately used for pre-rescue analysis and on-site treatment.

All response data is transmitted after being encrypted using the HTTPS protocol and sensitive information is further encrypted through an AES-256 algorithm. In addition, a connection log for each request and response is automatically stored in audit records to allow users or administrators to check access history later. As such, the electronic device 100 may provide a data interconnection structure that satisfies both quick emergency response and stable personal information protection.

In this embodiment, the electronic device 100 may implement an integrated operation structure integrated with wearable devices, smart home devices, external apps/services, medical information systems, and the like, in consideration of interoperability and scalability with various digital healthcare environments. As such, this enables evolving into a user-centric comprehensive health management platform and enhancing accuracy of biometric data collection, service versatility, and linkage with medical sites.

First of all, the electronic device 100 may include a function for interworking with wearable devices. For example, it may automatically receive data such as heart rate, activity amount, sleep time, and stress index from various wearable devices including smart watches, fitness bands, ECG measurement devices, and temperature patches, and integrate the received data into a health analysis model of the system. In this case, it may support communication protocols such as Bluetooth low energy (BLE), Wi-Fi, and near field communication (NFC), and may secure compatibility with standard data formats of major manufacturers (e.g., Apple HealthKit, Google Fit, Samsung Health platform, etc.) thereby flexibly supporting connection with devices of various brands.

In addition, the electronic device 100 may supplement health data in a non-invasive manner with less manual intervention through interconnection with smart home devices. For example, it may receive weight, body fat percentage and BMI data from a smart scale, collect sleep stage and quality information from a sleep monitor, and analyze the received data complementarily with data measured by a wearable device. In addition, interconnection with a smart blood pressure meter, an automatic medication reminder device, etc., may be included to implement a data collection environment covering all of the user's health indicators in daily life.

In addition, the electronic device 100 may provide an open API for linking a third-party app and service. The API is based on a RESTful structure and designed to allow bidirectional communication in which an external app or service requests some health data after user authentication (based on OAuth 2.0) or transmits analysis results to the system. For example, a diet management app, an exercise recording app, a psychological counseling app, etc., can automatically link with user data through the API and provide customized content to users. An access right of the API is set based on user consent, and the user can check data access history of the external app in the system.

Finally, the electronic device 100 may ensure compatibility with an electronic medical record (EMR) and an electronic health record (EHR) system used in a medical institution. To this end, it supports international medical information standards such as HL7 FHIR (fast healthcare interoperability resources), clinical document architecture (CDA), and DICOM, and enables affiliated medical institutions to directly link user health data collected by the system to a hospital's clinic information system or transmit a data package in a standard format when there is an emergency situation so that medical staff can immediately refer to the data. This compatible structure ensures continuity of care and maximizes the clinical utility of digital health data.

As described above, the electronic device 100 may implement a user-centric health information integrated ecosystem based on an organic connection with various hardware, software, and medical platforms, and may evolve into a next-generation healthcare system with both scalability and effectiveness.

In this embodiment, the electronic device 100 may include ethical considerations, measures for responding to legal regulations, and measures for securing accessibility in consideration of user diversity, in order to secure the accountability and reliability of an artificial intelligence-based health management system. This is an essential element for preventing development of technology from infringing on individual rights and enabling all users to use services without inconvenience.

First, the electronic device 100 may include a procedure for complying with an AI ethical guideline. In a process of performing prediction or analysis through an artificial intelligence model based on health data of a user, the system may automatically detect bias that may exist in training data of an algorithm and provide a warning notification to an administrator or developer when imbalanced learning is detected for a specific age, gender, race, disease group, etc. In addition, when processing data including sensitive information (e.g., genetic information, mental health information, etc.), the system may perform clear prior notice to the user and provide an option to deactivate the corresponding item depending on consent. Through such configuration, the system can secure a transparent and responsible AI operation structure.

In another embodiment, the electronic device 100 may ensure compliance with international privacy protection regulations. In consideration of global major laws such as General Data Protection Regulation (GDPR) in European Union, Health Insurance Portability and Accountability Act (HIPAA) in United States, etc., the system reflects user's right to delete data, access information, limit use for other purposes, etc., and provides a clear procedure for obtaining user consent on relevant items when collecting and utilizing data. In addition, the system is designed to record and provide storage history and usage purpose of collected data upon request from users.

In addition, the electronic device 100 may include a function for improving UX/UI for vulnerable groups, particularly a mode that guarantees accessibility for elderly people and disabled persons. For example, it may activate an increase in font size, a high-contrast color mode, a voice guide function, a simplified navigation structure, etc., according to user settings or automatic detection. In particular, screen reader compatibility design for visually impaired persons and text-based notification configuration for hearing-impaired persons may be used together. In addition, simple interfaces and large button configurations, repetitive reminders, etc., which minimize cognitive burden, may be applied as UX strategies targeting elderly users.

As described above, the electronic device 100 may be designed to satisfy the three principles of technical advancement, ethicality, legal legitimacy, and social inclusiveness together, which can lead to various practical advantages such as compliance with domestic and foreign regulations, user trust, and entry into public procurement markets in the long term.

In this embodiment, the electronic device 100 may include a premium subscription model and an incentive structure based on insurance company linkage for continuous operation of health management services and provision of customized high-value added services to users. Such configuration can realize revenue generation from data-based service while enhancing user participation motivation and also be linked with risk management and product diversification strategy of an insurance company.

First of all, the electronic device 100 may provide differentiated health management functions to paid users through a premium subscription model. For example, premium subscribers can access advanced analysis reports that are not accessible by basic users, predictive-based health trend analysis, disease risk prediction based on genes and lifestyles, and real-time 1:1 consultation with medical professionals or nutritionists, exercise coaches, etc. In addition, customized weekly health goals, personalized diet plans, and exercise plans are automatically generated and provided based on personal biometric data, and additional rewards (e.g., vouchers, points) according to achievement rates may be operated together. This model is very effective in terms of maintaining user loyalty, creating monthly regular revenue, and enhancing the premium value of the service brand.

As another example, the electronic device 100 may implement a structure in which user health data is securely shared with an insurance company under consent and an insurance premium discount benefit or a reward is provided based thereon. For example, if a user provides biometric data consistently for a predetermined period of time or more and faithfully carries out a health management goal (e.g., walking 10 thousand steps or sleeping at least seven hours), the insurance company classifies the user as a low-risk group and discounts the existing premium by a predetermined percentage or provides cashback to the user. To this end, the system is designed to automatically configure summary data for risk assessment based on a health index required by the insurance company and transmit it to the insurance company under the user's explicit consent. The data is transmitted through an encrypted channel, and the user can check the provision range and history thereof in the system at any time.

Such a premium and insurance linkage strategy is not limited to a simple healthcare system but serves as the basis for building a participatory incentive ecosystem in which users can receive substantial benefits by managing their health. As a result, the electronic device 100 can develop into a sustainable platform structure that provides substantial value to users and enhances the business performance of insurers and service providers.

In this embodiment, the electronic device 100 may include a function of providing an insurance premium discount benefit based on health data of the user through cooperation with an insurer. This configuration creates direct economic incentives in response to provision of user data and thereby encourage users to perform health management activities more actively. In addition, insurers can use user health data to evaluate risks more precisely and design personalized insurance products, and thus form a mutual benefit structure.

The electronic device 100 concludes a contract with an insurance company in advance, and when the contracted insurance company is connected to the system, the user may set whether to share health data for a specific insurance company through a UI screen. The consent may be divided into detailed items and can be withdrawn at any time. When the user explicitly consents to sharing of data, the system may transmit quantified health data such as activity amount, vital signs, sleep time, heart rate, exercise records, etc., in real-time or periodically to an insurance company API.

The insurance company may evaluate the health status of the user based on the received data and provide a discount or reward benefit at a predetermined rate from an existing premium according to the result of classifying the health level or risk group. For example, in case of a user who walks more than 8,000 steps every day or maintains an average sleep time of 7 hours or longer, it is determined that the health risk is low, so that a premium discount benefit may be applied. The electronic device 100 provides the discount application result or whether a reward is paid, which are delivered from the insurance company, to the user screen in a notification form, and the user experiences that his or her health management result is connected with a substantial benefit.

Security and privacy protection measures related to data sharing are operated based on enhanced policies. All data is encrypted with an AES-256 algorithm during transmission and storage, and information accessible by the insurer is limited to items agreed upon from a user. In addition, sensitive information may be provided in an anonymized form when necessary, and users can directly check data sharing history and access details on the system. An audit log may record a time point of data sharing, a receiving insurer, a shared range, and access information, and this record may be stored for at least one year.

Through such a structure, the electronic device 100 may integrate an ecosystem of cooperation with insurance companies into the health management platform to encourage healthy behavior of users and implement a data-based reward system connected to substantial economic benefits. As a result, this may serve as a key incentive factor for increasing service loyalty and continuously using the platform.

In this embodiment, the electronic device 100 may include a corporate wellness service and an AI-based data analysis insight report selling function for diversification of revenue models of healthcare services. This is a configuration that can realize expansion of high value-added-based B2B business targeting enterprise customers and affiliated institutions beyond simple personal health management services.

First of all, the electronic device 100 may provide a platform for integrated management of the health conditions of employees on an enterprise unit basis through an enterprise wellness program. An enterprise subscribes to the system to promote the well-being of its employees and employees voluntarily agree to provide biometric data (e.g., activity amount, sleep time, heart rate, stress index, etc.). The electronic device 100 visualizes this data to provide, based thereon, statistics of an in-house health index, distribution of stress, exercise participation rate by department, etc. to an enterprise manager as a weekly or monthly report. In addition, various internal incentive systems such as a reward program for achieving health goals linked with corporate welfare points, group challenge events, team-based ranking systems, etc may be operated together. Through this configuration, enterprises can expect effects such as maintaining employee health and improving productivity, reducing sick leave, and increasing institutional involvement, etc.

In addition, the electronic device 100 may implement a data productization model for providing an AI analysis-based data insight report to an affiliated institution for a fee. The collected health data is converted into an anonymized dataset by removing personal identifiable information, and based on this, an artificial intelligence analysis engine generates health trends, disease risk prediction statistics, and lifestyle pattern analysis reports according to elements such as age, gender, region, lifestyle habits, and chronic disease distribution. This report can be sold at a price to various affiliated institutions such as insurance companies, hospitals, research institutions, local governments, and wellness startups, and the configuration of the report may also be customized depending on the purpose of use (e.g., design of an insurance product, establishment of public health policy, training of a research model). The report is provided through API linkage or regular subscription, and main indicators are visualized in a dashboard form and can also be downloaded in a PDF/CSV format.

Such a configuration suggests that the healthcare service of the electronic device 100 evolves from a simple user-based health management tool to a B2B data-based platform business and may be used as a complex model in which fixed revenue (premium service) is harmoniously combined with variable revenue (data report sales). As such, the platform operator can realize a sustainable healthcare service based on a multi-layered ecosystem among individuals, enterprises, and institutions.

In this embodiment, the electronic device 100 includes a structure for commercializing user health data collected based on an AI analysis technique in the form of anonymized statistical information and providing it to affiliated institutions at a price. This function diversifies revenue models of healthcare platforms and operates as a B2B extension function that supports data-based decision making and product planning/research activities of public/private institutions.

The electronic device 100 may derive statistical insights based on the biometric data such as heart rate, sleep, activity amount, eating habits, and medication history collected from a user, configure them in a predetermined package form, and provide them to an affiliated institution. The corresponding data is subjected to anonymization processing for removing personal identifiable information (PII) so that direct identification of the user is impossible, and then converted into statistical information at a group level through clustering based on population statistics such as age, region, gender, and lifestyle.

The regular subscription model allows the affiliated institution to select a desired data type on a monthly or annual basis, and the system can continuously provide statistical data for the corresponding period, and the one-time purchase model enables purchasing of analysis results (e.g., sleep deprivation tendency among women in their twenties, distribution of high blood pressure risk groups in metropolitan areas) as a single set, and the data can be provided in standard formats such as JSON and CSV through an API, while the delivery method can be selected from real-time streaming or periodic batch transmission (e.g., once a week, once a month).

The health trend analysis may be an average change in heart rate by age group, a regional distribution of sleep quality, etc., the lifestyle pattern statistics may be a seasonal change in exercise habits and a weekday/weekend diet pattern, and the disease risk prediction statistics may be the ratio and characteristics of a group having high-risk factors for metabolic syndrome.

The health plan effect analysis may be a rate of change in the health index before and after participation in a specific health plan, and to protect security and privacy, the electronic device 100 may apply the following measures.

The data is provided only in an anonymized form and is designed so that the identity of any single user or small group cannot be identified under any circumstances, and the transmission paths are encrypted based on the HTTPS protocol, and if sensitive items are included, double encryption at the level of AES-256 or TLS may be applied.

The affiliated institution is restricted in access to the data based on its purpose of use and all terms of service are governed by contract with a clause prohibiting unauthorized resale or third-party provision of the data.

In addition, the electronic device 100 records all data requests, provision, and access history as an audit log to allow a user to check the flow of shared data later or track it when a dispute occurs.

Through such data productization structure, the electronic device 100 may realize B2B collaboration with various external partners such as research institutes, insurance companies, public institutions, and wellness enterprises, and secure a sustainable revenue base by converting the value of the platform into statistical assets. This model may serve as one of the core revenue axes in the digital healthcare industry.

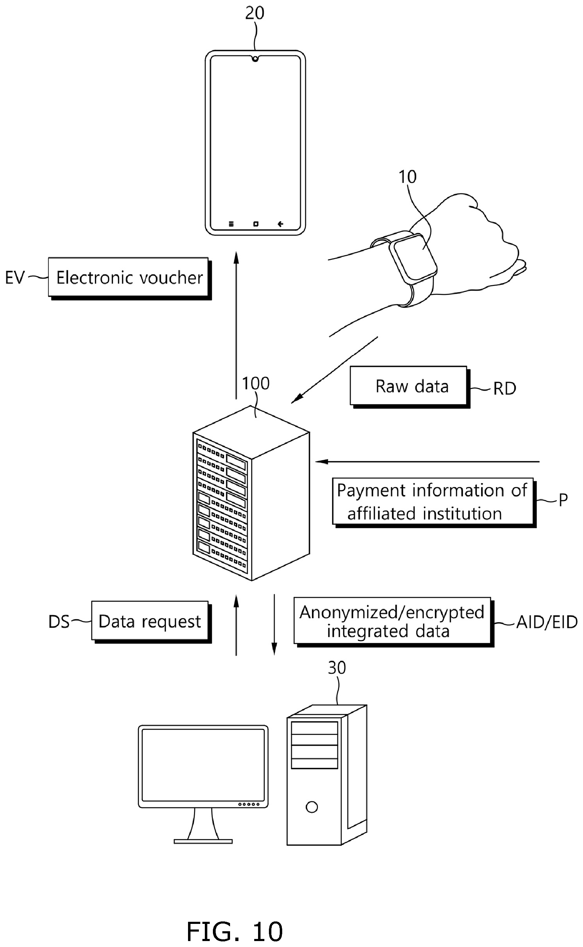

The operations of the user terminal 20, the affiliated institution terminal 30, and the electronic device 100 as described above may be described with reference to FIG. 10.

FIG. 10 is a diagram for describing operations of an electronic device 100, a user terminal 20 and an affiliated institution terminal 30 according to an embodiment of the present disclosure.

Referring to FIG. 10, the electronic device 100 may receive raw data RD from the user sensor 10. The electronic device 100 may transmit an electronic voucher EV to the user terminal 20.

The electronic device 100 may receive a data request DS from the affiliated institution terminal 30 and transmit anonymized AID or encrypted EID integrated data (ID) to the affiliated institution terminal 30 when payment information P of the affiliated institution is received from the external server or the affiliated institution terminal 30.

Here, the payment information P may be a donation deposit or a donation receipt, but is not limited thereto.

In addition to the above, according to various embodiments, the electronic device 100 may generate a customized health management plan HP for the user based on the raw data RD of the user (S50).

The electronic device 100 may transmit the customized health management plan HP to the user terminal 20 (S55). The user terminal 20 may output and provide the customized health management plan HP received from the electronic device 100 to a user (S60).

The electronic device 100 may transmit the generated customized health management plan HP to the user terminal 20, and the customized health management plan HP may be a health management plan HP for at least one of food F intake, exercise E, and sleep S.

The electronic device 100 may compare and analyze the measured data with a preset reference value to evaluate the current health status of the user.

For example, when the user's heart rate and respiratory rate D4 are higher than usual and body temperature D6 exceeds a normal range, the electronic device 100 may determine that the user is currently in a state of fatigue or stress. Based on this determination, the health management algorithm may recommend intake of food rich in water and vitamins F, or suggest a diet low in caffeine and easy to digest.

In addition, the long-term trend of collected biometric data may be analyzed to provide a customized exercise E plan for the user.

The electronic device 100 may reduce cardiovascular burden when the user's resting heart rate is continuously measured to be high by suggesting stretching and light cardio exercise E, instead of lowering the intensity of the exercise E, and may recommend more active strength exercise E or high-intensity interval exercise E when blood pressure D7 and heart rate are stable.

Sleep S is also managed based on the user's biometric data. When the user's biometric signal shows a specific pattern (e.g., an increase in heart rate and irregular breathing during sleep S), the electronic device 100 may generate a health management plan HP for customized activities before sleep S such as stretching, deep breathing, and meditation to create an environment conducive to sound sleep.

In addition, the electronic device 100 may detect a change in body temperature D6 during sleep S and generate a health management plan HP including recommendations for setting an environment such as room temperature or bedding.

In conclusion, the present embodiment actively utilizes biometric data to automatically generate a personalized health management plan HP for food intake F, exercise E, and sleep S, thereby actively managing the user's health status and effectively contributing to disease prevention and health promotion.

In addition, the electronic device 100 may generate dashboard DB information corresponding to the health management plan HP and transmit the dashboard DB information to the user terminal 20 so that it is displayed.

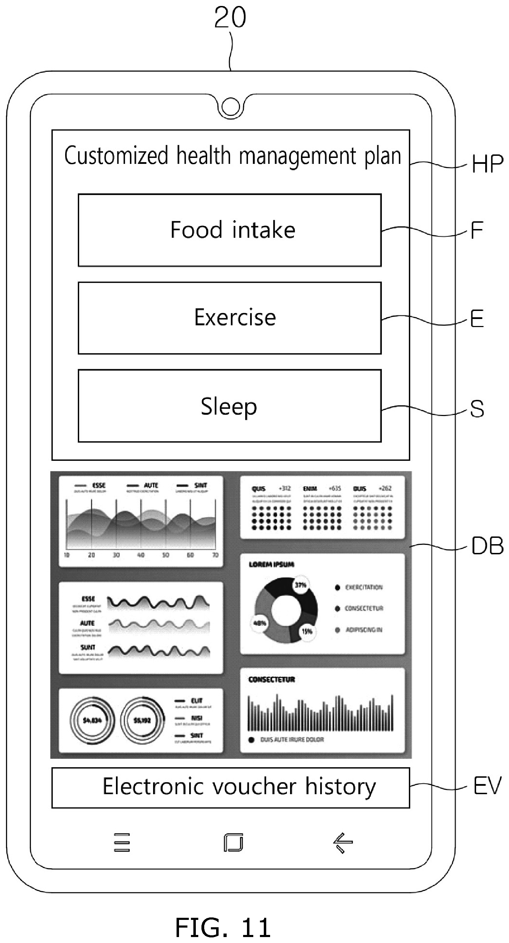

FIG. 11 is a diagram illustrating an example of a GUI for the health management plan HP and dashboard DB output by the user terminal 20 through the display to provide the same to the user according to an embodiment of the present disclosure.

Referring to FIG. 11, the user terminal 20 may output and provide a GUI based on data or information received from the electronic device 100, such as a customized health management plan HP, a dashboard DB, an electronic voucher EV detail, etc.

The electronic device 100 may apply a design of an elderly-friendly UI/UX, which includes a configuration for configuring the user interface to be intuitive and visually clear, providing a voice instruction-based input method, and outputting in multiple languages such as Korean, English, and Chinese, so as to improve usability of the elderly and digital access-vulnerable population. The electronic device 100 may be configured to automatically switch between default UI modes based on age groups, language settings, or device usage history of users, or adjust UI components (e.g., button size, font size, contrast ratio, etc.) through customized user settings.

The electronic device 100 and the user terminal 20 may provide a customized dashboard DB to intuitively and efficiently provide information to users. The corresponding dashboard DB represents real-time and past data such as heart rate, respiratory rate D4, body temperature D6, and blood pressure D7 in a visualized form so that users can check their biometric data at a glance. For example, the heart rate and blood pressure D7 are provided in the form of a real-time graph so that changes over time can be understood intuitively.

The dashboard DB clearly presents the health management plan HP and shows recommended food F intake, exercise E intensity and type, and sleep S management in separate sections. The section of recommended food F intake specifies a recommended diet and nutrient information based on current biometric data of the user, and may also provide recommendations such as an optimal time for eating during the day and an amount of water to drink.

The exercise E planning section provides information on recommended exercises E types, exercise E intensity, time and frequency based on the analysis results of the user's vital signs in icons or texts that are easy to understand, and also shows changes in the user's vital data after performing an actual exercise E. As such, the user can immediately visually check the effect of his/her exercise E on health conditions.

The sleep S management section analyzes the recent sleep S pattern of a user to visually evaluate the quality of sleep S, and intuitively provides whether deep sleep is achieved in an icon or a form of a sleep S score. In addition, it includes recommended activities before bedtime and guidance for setting up an optimal sleep S environment so that the user can easily recognize practical measures for improving sleep S.

Finally, the dashboard DB includes a health management goal achievement rate, whether there is any abnormality in vital signs, and notifications related thereto, to help users recognize and manage their own health status continuously. By providing such a dashboard DB, users can efficiently monitor their own health status and actively and persistently practice customized health management plans HP.

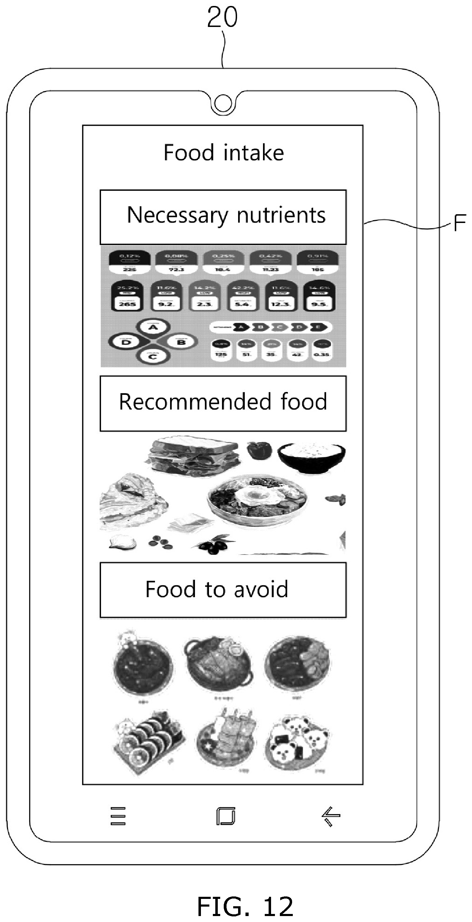

FIG. 12 is a diagram illustrating an example of a GUI for food F intake information output by the user terminal 20 and provided to the user through a display according to an embodiment of the present disclosure.

Referring to FIG. 12, the electronic device 100 may provide information related to intake of a personalized food F based on user biometric data. More specifically, the electronic device 100 may obtain raw data RD (biometric data) such as heart rate, blood pressure D7, blood sugar level, and body temperature D6 in real time or periodically through the user sensor 10 worn by the user.

The electronic device 100 may compare the raw data RD with a reference value or a normal range stored in advance to analyze the current health state and nutritional state of the user.

For example, when the user's blood sugar level is measured to be high, the electronic device 100 may extract necessary nutrients such as dietary fiber or low-sugar foods that help lower blood sugar and recommend them to the user.

The electronic device 100 may specifically recommend the recommended food F based on the user's biometric data. For example, if the blood pressure D7 of the user is measured to be high, the electronic device 100 may recommend a food F having a high potassium content (e.g., banana, spinach, sweet potato, etc.) as the recommended food F. In contrast, when it is determined that the condition of the user deteriorates in a state where the heart rate and blood pressure D7 are high, the electronic device 100 may specifically guide that stimulating foods F, such as coffee containing caffeine or green tea, should be avoided as foods F to be avoided.

In addition, the electronic device 100 may transmit information on food F consumption to the user terminal 20 in real time or periodically based on a result of analyzing biometric data of the user and provide the information visually on the display or deliver the information through a mobile app using push notification. As such, the user can selectively consume appropriate foods F according to his/her current health status and obtain an effect of improving health and preventing diseases by consuming foods F.

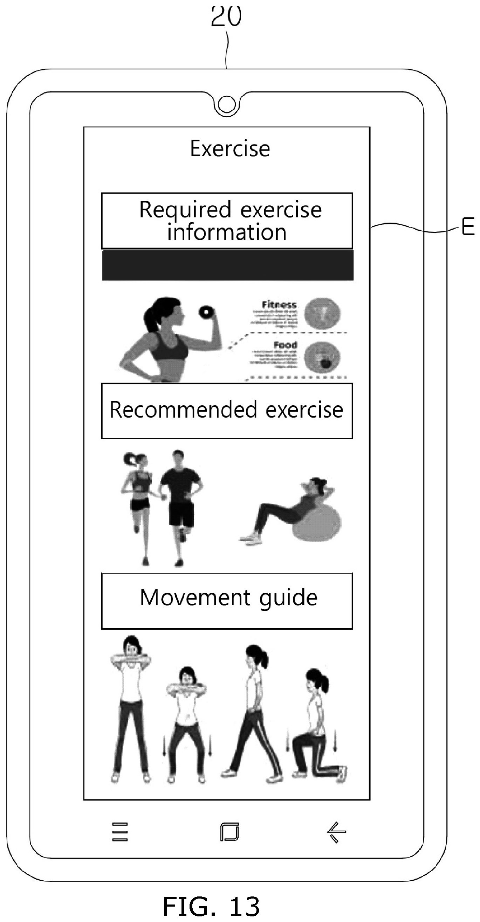

FIG. 13 is a diagram illustrating an example of a GUI for exercise E information output by the user terminal 20 and provided to the user through a display according to an embodiment of the present disclosure.

Referring to FIG. 13, the electronic device 100 may provide information related to a personalized exercise E based on raw data RD of the user, that is, biometric data. Specifically, the electronic device 100 may collect biometric data such as heart rate, oxygen saturation level, respiratory rate D4, body temperature D6, and activity amount through the user sensor 10 worn by the user.

The electronic device 100 may analyze the collected biometric data to evaluate a current physical condition and an exercise E performance ability of the user in real time. For example, when the heart rate of the user is higher than usual and oxygen saturation is low, the electronic device 100 may recommend a cardio exercise E (e.g., walking, light jogging, cycling, etc.) having a low intensity and smoothly supplying oxygen as a recommended exercise E. Meanwhile, it may recommend an exercise E such as high-intensity strength training E or high-intensity interval training (HIIT) when the heart rate, respiratory rate D4, and oxygen saturation of the user are measured stably.

In addition, the electronic device 100 may generate and provide necessary exercise E information based on the user's biometric data. For example, it may guide a recovery-type exercise E, such as stretching or yoga, to the user as the necessary exercise E when it is determined that the activity amount of the user is insufficient or the muscle fatigue degree is high.

In addition, the electronic device 100 may transmit information about the exercise E to the user terminal 20 to be displayed. For example, the user terminal 20 may provide a step-by-step description of a movements and a video for the recommended exercise E through a display or assist the user in accurately following the exercise E movements through voice guidance. It may continuously measure biometric data while the user performs the exercise E and provide feedback on whether the intensity and posture of the exercise E are maintained correctly in real time.

As described above, the electronic device 100 may provide customized necessary information related to exercise E and recommended exercise E based on biometric data of a user, and provide a movement guide for helping to perform accurate exercise E in real time, thereby maximizing the effect of the user's exercise E and contributing to physical health improvement.

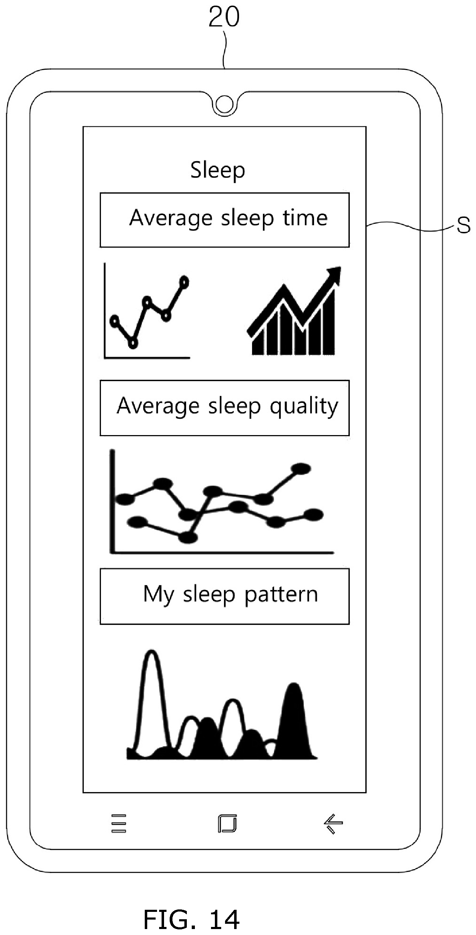

FIG. 14 is a diagram illustrating an example of a GUI for sleep S information output by the user terminal 20 and provided to the user through a display according to an embodiment of the present disclosure.

Referring to FIG. 14, the electronic device 100 may provide customized information related to sleep S of a user based on raw data RD, that is biometric data of the user. Specifically, the electronic device 100 may continuously collect biometric data such as heart rate, respiratory rate D4, movement level, and oxygen saturation during sleep S through the user sensor 10 worn by the user.

The electronic device 100 may analyze the collected biometric data to calculate an average sleep time S of the user. For example, the electronic device 100 may accurately detect a real sleeping time and a waking-up time of the user based on changes in the degree of movement, heart rate, and respiratory rate D4 of the user, measure the daily sleep time S, and provide the measured sleep time as a weekly or monthly average to the user.

In addition, the electronic device 100 may analyze the quality of the sleep S based on the user's biometric data and provide average sleep S quality information. For example, it may calculate a ratio of deep sleep S, light sleep S, and REM sleep S based on data such as stability of heart rate and respiratory rate D4, frequency and intensity of movement, and oxygen saturation level during the sleep S of the user, and display the quality of the sleep S in the form of a score or a graph by comprehensively evaluating the same.

In addition, the electronic device 100 may provide personalized sleep S pattern information of a user based on collected biometric data. For example, when it is detected that a user frequently wakes up at a specific time or has shallow depth of sleep S, it may provide personalized sleep S pattern information to the user based on such data and suggest a sleep S management plan for removing or improving factors interfering with sound sleep.

As described above, the electronic device 100 may provide customized sleep S information such as an average sleep time S, an average sleep quality S, and a personal sleep pattern S based on user biometric data, thereby helping the user efficiently manage their sleep S habits and improve the quality of sound sleep.

The provision of the raw data RD, management thereof, issuance and receipt of an electronic voucher EV, and information on food F, exercise E, and sleep S obtained based on the raw data RD may be implemented through an application implemented in the user terminal 20.

In addition, a service in which the user can pay for and receive recommended foods F, recommended health management products, etc., using an electronic voucher EV provided to the user terminal 20 may be implemented through the application.

The electronic device 100 may provide an electronic voucher EV as a reward differentiated according to whether the user achieves a health improvement goal with respect to the collection and provision of biometric data.

For example, when the biometric data of a user such as blood pressure or blood sugar is close to or reaches a preset health target value, the electronic device 100 may evaluate this and provide various electronic vouchers EV, such as a voucher for subscribing to a health management program, a discount voucher for a fitness center, or a voucher for purchasing a health-related product, based on the degree of achieving the goal.

In another embodiment, the electronic device 100 may provide a special event electronic voucher EV to a user who provides biometric data in real time during a specific time period or a specific event.

For example, when participants of a sports event such as a marathon or bicycle race provide their biometric data such as heart rate, body temperature, and respiratory rate to the electronic device 100 in real time during an event, the electronic device 100 may accumulate and analyze health data specialized for the event based on the provided biometric data, and then provide users with dedicated discount vouchers EV or electronic vouchers EV in commemorative free exchange coupons that can be used at an official commemorative online store after the event.

According to various embodiments of the present disclosure, the present disclosure relates to an AI-based green welfare card system, and more particularly, to an electronic device 100 that generates predicted data such as body temperature and blood pressure by analyzing biometric data collected through a wearable device using an artificial intelligence-based data prediction model, provides the same to an affiliated institution after anonymization and encryption, and provides an electronic voucher in proportion to the amount of data provided and a customized health management plan to a user.

The electronic device 100 receives biometric data collected from the wearable device of a user. The collected data is raw data such as heart rate, respiratory rate, and movement, and the corresponding data may be transmitted to the electronic device 100 periodically or in real time.

The electronic device 100 uses a convolutional recurrent neural network (CRNN) model to predict an index such as body temperature and blood pressure based on the received biometric data. The CRNN is configured by combining a CNN layer for extracting visual features with an RNN layer for recognizing patterns according to time flow, and can effectively analyze time-series data such as biometric data to generate accurate prediction results.

The electronic device 100 dynamically pseudonymizes the predicted biometric data to protect personal information and securely processes the data by applying an AES-256 encryption algorithm. The data is structured according to the HL7 Fast Healthcare Interoperability Resources (FHIR) standard, and provided to affiliated institutions such as hospitals and insurance companies. The HL7 FHIR is an international standard for exchanging medical data, which simultaneously satisfies interoperability and security.

The electronic device 100 complies with domestic and foreign major regulations such as the GDPR, HIPAA, and Personal Information Protection Act in order to protect data and operate ethical AI, and performs transparent and responsible information processing according to an AI ethics guideline. In addition, it introduces post-quantum cryptography technology based on Kyber and Dilithium to secure long-term data security by responding to security threats in a quantum computing environment.

The electronic device 100 is configured to maximize usability by providing an intuitive interface and a multilingual support environment for the elderly and information-vulnerable groups, and extend services to users of various ages and language regions.

Therefore, the electronic device 100 according to the present disclosure is an integrated welfare management system configured in a balanced manner with respect to AI analysis, standard-based data provision, reward systems, user customized services, security and accessibility, thereby realizing a next generation platform covering medical, welfare and financial areas.

In addition, the present disclosure relates to an artificial intelligence-based data prediction system for predicting the body temperature and blood pressure of a user by using biometric data, and more particularly, to an electronic device 100 that generates high-precision prediction values by inputting heart rate and respiratory rate data based on a CRNN model which is combined CNN and RNN model, integrates input data collected from various wearable devices, and continuously improves accuracy through periodic retraining.

The electronic device 100 collects heart rate, respiratory rate, oxygen saturation (SpO2) and heart rate variability (HRV) data from the wearable biosensor of a user. The devices used here include wearable devices from various brands such as Apple Watch, Samsung Galaxy Watch, Garmin, Fitbit, and Suunto, and differences in data format between sensors are standardized through a standard interface.

The electronic device 100 uses the CRNN model to predict body temperature and blood pressure based on collected biometric data.

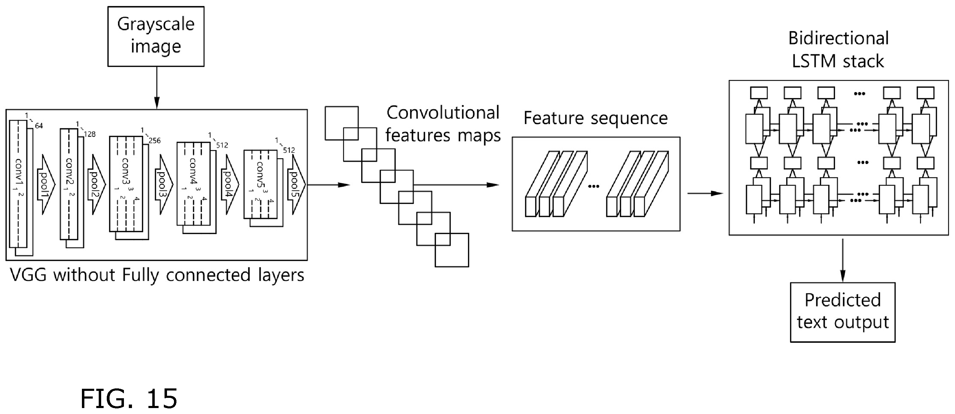

FIG. 15 is a block diagram illustrating the structure of a CRNN according to an embodiment of the present disclosure.

Referring to FIG. 15, a convolutional recurrent neural network (CRNN) is an artificial intelligence model that extracts features of visual or time-series data using a CNN structure and generates prediction values by considering the order of time using an RNN structure. In the present invention, attention mechanism is combined with the CRNN to improve the accuracy of prediction by reflecting the importance of each input point in time. The model predicts body temperature and systolic/diastolic blood pressure by paying attention to changes in patterns of heart rate and respiratory rate, and the average prediction accuracy was measured as more than 92%.

The electronic device 100 is trained based on an anonymized biometric dataset of more than 10 million pieces of data. The corresponding dataset includes raw data collected from medical institutions and wearable devices, and model training is performed in a supervised learning manner through labeling by medical experts. The electronic device 100 has reduced the error rate by 15% or more compared to existing prediction models, and processing time is shortened by 30%.

The electronic device 100 performs retraining at a predetermined period of time every month and prevents quality degradation by reflecting newly collected data in the form of separate training without accumulating the new collection data to an existing model during retraining. In this process, z-score outlier removal is performed to maintain data quality of 95% or more. Re-training is performed through an automated pipeline, and each model version is managed as a version in an internal storage.

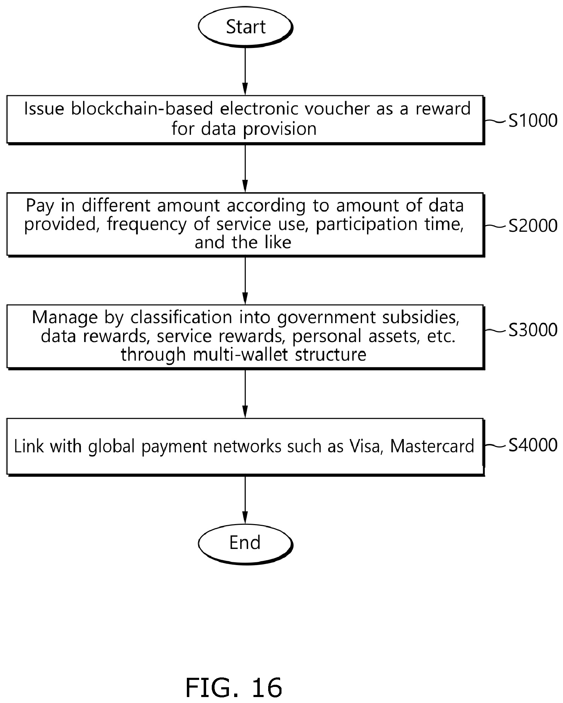

FIG. 16 is a flowchart illustrating a process example for implementing a multi-wallet system using a blockchain voucher network and explaining an actual payment example according to an embodiment of the present disclosure.

Referring to FIG. 16, the electronic device 100 issues a blockchain-based electronic voucher as reward for data provision (S1000). The electronic voucher is paid in different amounts according to the amount of data provided, frequency of service use, participation time, and the like (S2000), and managed by being divided into government subsidies, data rewards, service rewards, personal assets, etc., through a multi-wallet structure (S3000). The electronic device 100 connects with global payment networks such as Visa and Mastercard so that the voucher can be used for actual consumption (S4000).

The electronic device 100 sets a long-term goal for performance improvement to achieve a mean absolute error (MAE) of 0.1 °C in body temperature prediction and an MAE of 3 mmHg (systolic)/2 mmHg (diastolic) or less in blood pressure prediction by 2030. This goal is used as a quantitative criterion when retraining the model so that if it fails to maintain a certain level of performance, subsequent tuning procedures are automatically performed.

As a result, the present invention can realize a biometric data-based prediction system that predicts high-precision health indicators based on various biometric input information, simultaneously secures the accuracy and processing speed of a model, and improves long-term usability and reliability through retraining and data quality management.

The present disclosure relates to a data security technology for securely protecting user biometric data and thoroughly guaranteeing the privacy of users in providing related services. In particular, it is an electronic device 100 that integrates advanced encryption techniques such as dynamic pseudonymization, homomorphic encryption, post-quantum cryptography, zero-knowledge proof, etc., to improve protection levels throughout the stages of collecting, storing, analyzing, transmitting, and utilizing data.

The electronic device 100 collects biometric data and then immediately pseudonymizes the user identification information. The pseudonymization is performed in a dynamic pseudonymization manner of generating new identifiers in real time rather than using a fixed mapping scheme. In this case, the mapping table is distributedly stored on multiple nodes instead of one central server to prevent exposure of all mappings due to a single breach. As a result, the possibility of re-identification is reduced to less than 0.01%, and the pseudonymized information is used only for internal processing and analysis.

When sensitive data needs to be processed or shared with an external entity, the electronic device 100 applies homomorphic encryption that can process data without decryption. Homomorphic encryption allows operations such as addition and multiplication even in an encrypted state, so health indicators may be calculated or anomalies detected in an encrypted state under cooperation with an affiliated institution. This method shows about 20% improvement in processing speed compared to conventional methods, enabling high-performance analysis while protecting privacy.

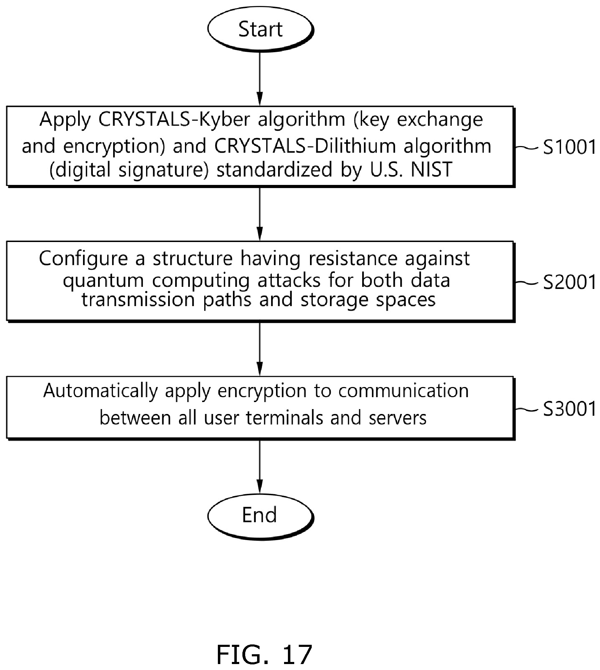

FIG. 17 is a flowchart illustrating a post-quantum cryptography process according to an embodiment of the present disclosure.

Referring to FIG. 17, the electronic device 100 introduces post-quantum cryptography in order to secure long-term security. Specifically, a CRYSTALS-Kyber algorithm (key exchange and encryption) and a CRYSTALS-Dilithium algorithm (digital signature) standardized by the U.S. NIST are applied (S1001), and a structure having resistance against quantum computing attacks is configured for both data transmission paths and storage spaces (S2001). The corresponding encryption is automatically applied to communication between all user terminals and servers (S3001).

The electronic device 100 additionally uses a zero-knowledge proof (ZKP) technique to prevent user information from being exposed in the process of issuing and using welfare vouchers. The user can prove eligibility to use the voucher and have it verified without disclosing specific personal health information or personal identification information. This prevents personal information from being provided to affiliated vendors or third parties during the voucher redemption process.

The electronic device 100 is designed to comply with laws and regulations regarding data protection in various countries. Data of Chinese users are stored on a local server and restricted from being transmitted abroad according to the Cybersecurity Law of the People's Republic of China (PRC). For Japanese users, Act on the Protection of Personal Information (APPI) is complied with to prevent collection for purposes other than those intended and limit data collection to minimum items. The electronic device 100 is configured to automatically apply policies for processing data based on user country information and request re-consent when switching between countries.

As described above, the electronic device 100 according to an embodiment of the present disclosure is designed in a manner that data security and privacy protection are enhanced, thereby providing a high-reliability secure framework having regulatory compliance capability for safe use of sensitive information as well as global service expansion.

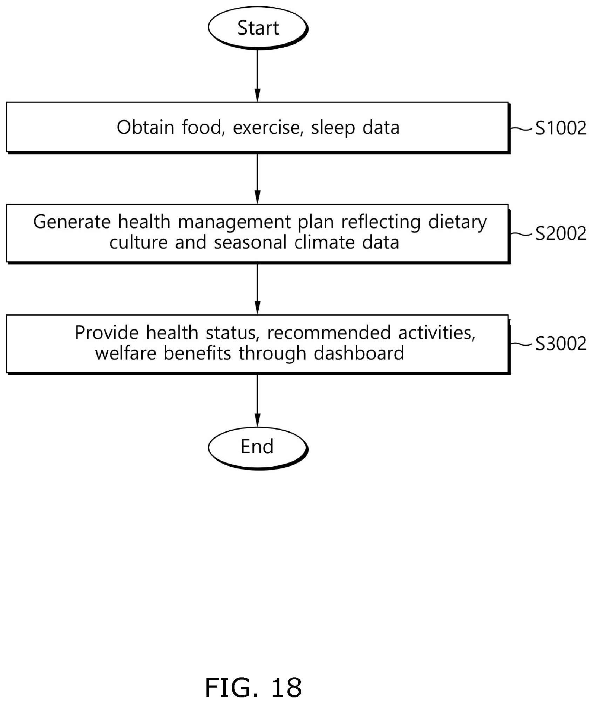

FIG. 18 is a flowchart for providing a health management plan based on Korean dietary culture and climate data according to an embodiment of the present disclosure.

Referring to FIG. 18, the electronic device 100 analyzes collected and predicted biometric data to provide a customized health management plan for the user. The electronic device 100 identifies data such as food, exercise, and sleep (S1002), and the health management plan is designed by reflecting Korean dietary culture and seasonal climate data by the electronic device 100 (S2002). Health status, recommended activities, welfare benefits, and the like may be intuitively checked through a dashboard generated by the electronic device 100 (S3002).

The present invention relates to a system for providing user biometric data and welfare benefits based thereon, and more particularly, to an electronic device 100 that calculates differentiated electronic vouchers based on the amount of user data provided and usage performance thereof, separately manages the corresponding voucher using a multi-wallet system, includes use restriction and real-time spending control, and stores all transaction records in a blockchain to prevent forgery.

The electronic device 100 calculates the voucher amount based on the data providing performance of the user. The basic algorithm is configured to linearly increase the voucher amount according to a daily data providing volume, for example, when more than 1,000 pieces of biometric data are provided in one day, the voucher amount increases according to a reference unit price. In addition, an additional incentive of 10% bonus amount is automatically paid to users whose monthly welfare service use amount exceeds KRW 1 million. Such an algorithm is applied to a user account by being linked with a real-time statistics system.

The electronic device 100 also provides a separate reward for providing data on a specific event. For example, if the user who participates in a health promotion activity such as a walking campaign or marathon event submits biometric data of a specified type during an event period, an event-specific voucher of up to KRW 50,000 is separately issued. The event voucher can be used within a designated period and usage history thereof is recorded separately.

The electronic device 100 manages the user's electronic voucher by dividing it into a multi-wallet system. The multi-wallet system is divided into a restricted wallet for government subsidy use and a free wallet in which information provision reward or autonomous contribution is accumulated. The restricted wallet can be used only at specific stores or product groups, while the free wallet may be utilized as desired by the user. Switching between wallets is allowed to a limited extent, and switching conditions are set according to a system administrator or policy.

The electronic device 100 performs real-time control of the user voucher spending by being linked to a Marqeta payment platform. When a request for voucher payment is made by the user, store information and product information are immediately checked in the corresponding transaction, and it is determined whether or not the restricted wallet can be used based on Merchant Category Code (MCC). For example, government subsidy wallets allow only medical care, health foods, public transportation, etc., and automatically block payments from other categories.

The electronic device 100 records the issuance and use history of the electronic voucher on a blockchain. This record does not rely on a centralized server but is maintained equally in several nodes according to distributed ledger technology, which can prevent falsification at the source. The main information such as the voucher identification number, issuance time, use time, expenditure details, MCC code, etc., are all stored after being hashed, and access records may be provided to users when necessary.

As a result, the electronic device 100 may provide a welfare voucher operation system that controls use of the voucher and simultaneously ensures security and transparency while providing data-based rewards in real time, thereby improving both the effectiveness of public welfare policies and user participation rates.

The present invention relates to an electronic device 100 that establishes a customized health management plan based on the user's biometric data and dietary culture information, and provides personalized services by reflecting cultural diversity and real-time biometric responses.

The electronic device 100 periodically analyzes the biometric data collected through the wearable device of the user. The biometric data includes heart rate, respiratory rate, sleep cycle, activity amount, etc., and is collected in real time to be reflected in a health management plan at an interval of one hour. The electronic device 100 automatically generates a daily plan optimized for the physical condition of the user by dynamically adjusting meal recommendation time, food intake list, sleep and exercise time, etc., according to changes in analyzed biometric data. This dynamic plan update has an effect of improving user satisfaction by more than 30%.

The electronic device 100 integrates regional dietary culture and environmental data in establishing a customized health management plan. It suggests foods and exercise patterns suitable for the user's location and registered cultural background by considering nutritional recommendation standards of various regions such as America, Europe, and Asia, accessibility to ingredients, climatic characteristics, etc. For example, in case of users from Asian countries, diets using fermented foods such as kimchi and doenjang are reflected, and Mediterranean diets based on olive oil are suggested for users from European countries.

The electronic device 100 reflects cultural diversity elements for elderly people and multicultural family members in establishment of the plan. To this end, it automatically filters food compositions that avoid foods ingredients difficult to chew for elderly people and consider religious dietary restrictions (e.g., halal or kosher) of multicultural families. In addition, the electronic device 100 customizes and designs the user interfaces (UI) and user experiences (UX) so as to minimize language and cultural barriers. The large fonts and voice guidance functions to consider poor vision or difficulty in operation of elderly people are included, and provides multilingual environments such as English, Chinese, Vietnamese, Arabic, etc., in addition to Korean for multicultural users are provided.

The electronic device 100 may generate and update a daily health management plan for each user by integrating such cultural elements with the real-time biometric information reflection function, and provide feedback on the implementation of the plan in the form of a visualized dashboard to offer a personalized welfare experience. The plan data is also linked to a voucher issuing system, a risk detection and notification system, and a collaborative treatment recommendation system with a affiliated medical institution within the system.

Therefore, the electronic device 100 according to the present invention may function as an integrated customized health management system that flexibly responds to cultural differences and biological changes while efficiently supporting achievement of a user's health goal.

The present invention relates to a health management data providing system that provides user biometric data to various affiliated institutions in a customized manner according to the purposes, enhances processing speed and security, and considers regional characteristics and industrial utilization value. In particular, the present invention relates to an electronic device 100 which subdivides a provided data package according to requirements of various industries such as hospitals, insurance companies, pharmaceutical companies, etc., optimizes real-time API performance, and simultaneously satisfies international standards and quantum security algorithms.

The electronic device 100 configures diet and exercise plan data for establishing a health management plan based on the user's dietary culture and regional data and providing it to an affiliated institution. For example, in case of Korean users, the frequency and amount of intake of traditional ingredients such as kimchi and doenjang are extracted, and functions for automatically adjusting outdoor exercise time and place by linking real-time air quality data of Seoul are included. Such region-specific data is used not only for personalized health management but also as important basic data for institutional health analysis.

The electronic device 100 differentiates the data to be provided by each affiliated institution, flexibly responding to industry needs. Hospitals are provided with a data package designed to be in a structure linkable with electronic medical record (EMR) systems in compliance with the HL7 FHIR (Fast Healthcare Interoperability Resources) R4 standard. These packages include heart rate, respiratory rate, and blood pressure data measured at one-second intervals, which can be utilized for real-time emergency response and critical care monitoring. Meanwhile, insurance companies are provided with statistical data-including average blood pressure by age group, chronic disease incidence rates, and lifestyle-based risk indices-to facilitate risk assessment and premium calculation.

The electronic device 100 supports high-speed and large-volume data transmission based on a RESTful API structure. The server is designed to process more than 1,000 requests per second, and the average response speed is maintained within 100 ms. The API is accessed under an authentication scheme based on OAuth 2.0, and the data structure follows a format of FHIR R4 standard based on JSON.

The electronic device 100 applies post-quantum cryptography for security of data transmission and storage. Specifically, it uses CRYSTALS-Kyber (encryption) and CRYSTALS-Dilithium (signature) algorithms to ensure that sensitive health information transmitted to hospitals and insurance companies is safe even in future quantum computer environments.

The industrial applicability of the electronic device 100 is demonstrated through specific examples. An insurance company applied blood pressure and exercise pattern data collected from the system to a risk assessment algorithm, thereby improving accuracy by more than 18% compared to the existing method and ensuring fairness in premium calculation. A hospital linked real-time heart rate data with an emergency prediction system, thereby increasing the accuracy of predicting occurrence of an emergency situation by more than 25%, and also contributing to optimization of ICU bed arrangement.

Therefore, the electronic device 100 according to the present invention may implement a platform optimized for collaboration with an affiliated institution and customized data interconnection through a highly advanced structure in terms of regional characteristics, industry-specific requirements, real-time processing performance, standard compliance, and security.

The electronic device 100 may receive raw data including heart rate and respiratory rate from the user sensor through a communication interface, obtain predicted data on body temperature and blood pressure by inputting the raw data into the CRNN-based data prediction model, generate integrated data by anonymizing the raw data and the predicted data using dynamic pseudonymization, transmit the same to an affiliated institution terminal in accordance with the HL7 FHIR standard, and issue an electronic voucher proportional to the amount of provided data based on blockchain technology to transmit the same to the user terminal.

The electronic device 100 may be integrated with global payment networks such as Visa and Mastercard, thereby being usable like a check card worldwide.

The electronic device 100 may perform a dynamic pseudonymization scheme of periodically updating the identifier of the user. The electronic device 100 may store or transmit the data using AES-256 encryption.

The electronic device 100 may comply with the HL7 FHIR (Fast Healthcare Interoperability Resources) standard during the data transmission process.

In the electronic device 100, CRNN model may combine a CNN and an RNN to so that simultaneously learn spatial and temporal patterns in heart rate data and the respiratory rate data.

The electronic device 100 may include a multi-wallet system including a restricted wallet for storing government-issued welfare vouchers and a free wallet for storing information provider reward or discretionary funds.

The electronic device 100 may be protected from quantum computing threats using a post-quantum cryptography technology including CRYSTALS-Kyber and CRYSTALS-Dilithium.

The electronic device 100 may provide a user interface including voice instruction input, large button UI, and multi-language support.

More specifically, the electronic device 100 includes various security and privacy protection technologies for secure collection, processing, and transmission of user data. In particular, the electronic device 100 integrates a dynamic pseudonymization scheme, high-strength encryption, standardized data transmission, advanced prediction models, wallet systems, global payment integration, and elderly-friendly UI functions to implement a practical and scalable AI-based welfare system.

The electronic device 100 performs a dynamic pseudonymization manner of periodically updating the identifier of the user to strengthen personal information protection. This is a method of replacing unique identification information of the user with an arbitrary pseudonym identifier at regular intervals and distributing the corresponding mapping table across a separate security server to minimize re-identification risk.

The electronic device 100 stores and transmits the collected biometric data and related information by applying an AES-256 encryption algorithm to the biometric data and related information. As such, this prevents data alteration or falsification and can respond to hacking threats that may occur during network transmission.

The electronic device 100 ensures interoperability and data consistency by complying with the HL7 Fast Healthcare Interoperability Resources (FHIR) standard when performing data interconnection with affiliated institutions such as hospitals or insurance companies. The standard enables structuring, exchanging, and searching of medical information to enable interconnection with various systems.

The electronic device 100 may include a convolutional recurrent neural network (CRNN) model.

The corresponding model combines a CNN and an RNN to simultaneously learn spatial and temporal patterns based on heart rate and respiratory rate data. As such, this enables the prediction of vital signs, such as body temperature and blood pressure, with high accuracy.

The electronic device 100 manages welfare vouchers and autonomous user funds separately through a multi-wallet system. One or more restricted wallets store government-issued welfare vouchers, and one or more free wallets may store information provision rewards, service use rewards, personal assets of the user, etc.

The electronic device 100 adopts a post-quantum cryptographic algorithm configured with CRYSTALS-Kyber and CRYSTALS-Dilithium for long-term security of sensitive information. As such, this enables data to be protected safely even in the quantum computing environment.

The electronic device 100 may manage the usage of a restricted wallet based on merchant category code (MCC), and restrict the welfare voucher to be used only for designated purposes such as grocery stores, hospitals, or pharmacies. As such, this can prevent unintended use of public funds.

In addition, the electronic device 100 is integrated with a global payment network such as Visa and Mastercard to support use of the voucher by users worldwide through a physical card or digital payment method.

Finally, the electronic device 100 provides a user interface including voice instruction recognition, large button UI, and multi-language support to increase digital accessibility of elderly users and low-income users. As such, even the information-underprivileged can easily use the system.

Therefore, the electronic device 100 of the present disclosure may provide an integrated welfare infrastructure that is excellent in terms of data protection, system security, user convenience, industrial compatibility, and scalability.

The electronic device 100 may be implemented as follows, including a customized health management plan according to various user conditions and situations, reward provision, wallet use, and secure communication function.

The electronic device 100 may collect and analyze health state data for an elderly user to suggest a customized diet. For example, when heart rate of 90 bpm and blood pressure of 140/90 mmHg are collected from the elderly user aged 70 years old, the electronic device 100 may recommend a recipe including water dropwort in a low-sodium diet that limits sodium intake to help manage blood pressure.

The electronic device 100 may also provide a reward for the participant of a specific event. For example, when a user who participates in a marathon event provides heart rate data for one hour, an electronic voucher worth KRW 30,000 may be automatically issued and provided to the user based on the provision of the corresponding data.

The electronic device 100 may suggest a health management plan that reflects the culture and living environment of global users. For example, an American user may be recommended exercise-oriented health plans, a European (EU) user may be provided with elderly stress management-oriented plans, and an Asian user may be provided with nutrition intake plans based on multicultural diets.

The electronic device 100 may classify payments according to various use purposes through the multi-wallet system. For example, a limited wallet configured with government subsidies can be used at pharmacies for welfare funds, and a free wallet accumulated as reward for providing information allows users to freely purchase items at general stores.

The electronic device 100 may apply post-quantum cryptography technology to enhance data security. For example, when user health data is transmitted to the server, encryption based on CRYSTALS-Kyber and CRYSTALS-Dilithium may be applied so that secure communication can be guaranteed even in a future quantum computing environment.

In addition, the electronic device 100 may perform reward linkage according to user behavior through interconnection with an insurance company. For example, when a user maintains an average of more than 8,000 steps per day for six months, the electronic device 100 interconnected with an insurance company system may apply a premium discount of 10% to the user or notify the corresponding incentive through notification.

The electronic device 100 according to the present disclosure has a technical effect of integrating welfare linkage, regional specialized services, and blockchain-based electronic voucher functions to simultaneously satisfy publicness and security unlike existing wearable-based health management systems. For example, commercial platforms such as Fitbit or Apple Health focus on collecting and analyzing health data but have limitations in integration with social infrastructure data such as linking with welfare policies, reflecting Korean dietary culture, and linking with insurance company incentives.

In contrast, the present invention provides differentiated effects over existing technologies through, first, a health management plan reflecting Korean dietary culture and environment data; second, dynamic pseudonymization and security design based on AES-256, post-quantum cryptography (Kyber/Dilithium); third, voucher issuance and use history verification based on blockchain technology; fourth, multi-wallet system for separately managing government subsidies and user assets; fifth, integration with global payment networks such as Visa and Mastercard, and sixth, **high accessibility user interface (UI/UX) for elderly people and information-vulnerable groups**.

In addition, the present invention applies an encryption technology designed to maintain security even in a quantum computing environment, thereby securing preemptive response capabilities against information protection threats that may occur in the future. In addition, by operating restricted wallets and free wallets separately, it is possible to implement flexible user-customized services while maintaining a balance between policy-based fund flows and personal autonomous consumption.

As a result, the present invention enables implementation of an electronic device with excellent technical, industrial, and policy scalability through a highly advanced platform structure integrating welfare, finance, healthcare, and information protection, and can realize a public-private convergence-type AI healthcare system that has not been provided in the existing market.

The existing representative healthcare system, the Fitbit, is configured to focus on simply collecting and visualizing biometric data, and although it has a function of receiving information such as heart rate and activity amount from the user and performing real-time monitoring, it does not provide an in-depth analysis based on the data or a precise health index prediction function. Meanwhile, the present invention includes an artificial intelligence function capable of predicting quantitative health indices such as body temperature and blood pressure in real time from raw data such as heart rate and respiratory rate by using a CRNN model combining a CNN and an RNN.

In addition, there is a clear difference in terms of personal information protection. Although Fitbit uses general encryption methods to protect data, the present invention minimizes the possibility of re-identification of personal information by applying dynamic pseudonymization techniques that update user identifiers in real time and post-quantum cryptographic algorithms (Kyber, Dilithium) for future quantum computing era.

In terms of functionality, the Fitbit is limited to health tracking functions in a single device, whereas the present invention provides government welfare vouchers in an electronic form through a multi-wallet structure linked with a welfare system and adopts a structure capable of providing information provision rewards and freely managing personal assets. This enables a structure that simultaneously satisfies public interest and individual convenience.

Even at the regional specificity level, the present invention shows a clear technical distinctiveness. While Fitbit operates its functions based on common data around the world, the present invention can provide health management plans in consideration of Korean dietary culture and climate environment, and has scalability to provide customized health services for users by region in connection with various country-specific environmental information in the future.

In payment linkage, the Fitbit provides a limited self-platform-centric payment function, but the present invention is configured to ensure voucher usability anywhere in the world through linkage with global payment networks such as Visa and Mastercard. As such, this enables securing substantial universality and accessibility of digital welfare cards.

The electronic device 100 of the present invention is designed to comprehensively consider multilayered regulatory requirements such as personal information protection, financial regulations, accessibility, and ethical artificial intelligence, thereby simultaneously securing the technical reliability and legal compliance necessary for global service provision.

First of all, in terms of personal information protection, the electronic device 100 provides an interface for allowing a user to withdraw his or her data provision consent at any time, reflecting Article 7 of the EU GDPR, and implements a technical procedure for automatically suppressing user data within 24 hours after receiving a corresponding withdrawal request. In addition, by storing Chinese user data on servers within China according to the data localization requirement of the Cybersecurity Law of the People's Republic of China, and complying with a minimum data collection policy that complies with Japanese APPI regulations, it is possible to comply with legal requirements of major global markets.

In terms of the fintech function, it is ensured that the system including an electronic wallet and a voucher payment function complies with the EMV standard and the PCI DSS which are international standards for processing financial data, thereby making the system suitable for linkage to financial institutions and card companies. In particular, even when sensitive funds such as government welfare vouchers are included, secure payment processing can be performed technically.

In terms of accessibility, the present invention is designed to comply with the Web Content Accessibility Guidelines (WCAG), and provides voice instructions, large-sized UI buttons, multilingual support functions, etc., so that elderly people, visually impaired persons, and information underprivileged can use the system. This increases its value as a social technology capable of evenly expanding user groups.

Meanwhile, by introducing a zero-knowledge proof (ZKP) technology for user privacy protection, it is designed introduced to design the electronic voucher system such that authentication can be performed without revealing the user's sensitive information of the user when using an electronic voucher. This is technically linked with a blockchain technology-based anonymous reward system.

Finally, the present invention includes an algorithm bias detection system for ethical AI implementation, which detects and corrects decision-making bias in real time according to gender, age, etc., thereby maintaining fairness of AI prediction results at a level of 98%. As such, this prevents potential discrimination problems that may be caused by artificial intelligence based healthcare/welfare systems, and ensures user confidence.

The electronic device 100 of the present invention is designed to be widely applicable in public and private welfare systems, and has a technical structure capable of providing substantial industrial and commercial value.

In terms of public welfare, the system can provide customized health management and welfare voucher services for elderly people through a system link with the National Health Insurance Service, thereby making it possible to provide substantial support to those who are in medical blind spots. In particular, since it is possible to design preventive welfare programs linked with local governments and public institutions based on user interfaces specialized for elderly people and continuous collection of health data, its usefulness as a means of public policy is also high.

Private partnership scalability is also a key competitive element of the present invention. By providing a system that can be used in actual consumption sites through voucher partnerships with pharmacies, fitness centers, food stores, etc., user accessibility and satisfaction may be improved at the same time. In particular, user satisfaction due to an expanded voucher use site may increase by up to 50% or more compared to the existing service, which becomes a major factor for increasing the service retention rate.

In terms of data economy, the present invention has a structure that can provide an anonymized biometric data-based dataset to pharmaceutical companies, insurance companies, healthcare enterprises, etc., for a fee. The data productization may stably secure millions of high-quality health data per year and generate profits of over KRW 10 billion annually, indicating that the technology is not only a simple infrastructure but also a profit model in the industry.

In addition, the present invention is designed to comply with major national health and personal information protection regulations such as HIPAA in the United States, GDPR in Europe, and APPI in Japan, and has an advantage of being applicable to financial and welfare industries around the world including North America, Europe, and Asia on the same platform through post-quantum cryptography and global payment network integration functions. In particular, it can be linked to a global insurance company to provide benefits such as insurance premium discounts based on user's health data and refinement of a risk assessment model, thereby providing practical incentives for expansion of partnership.

In the case of a pharmaceutical company, large-scale biometric signal-based lifestyle and drug response data collected through this system can be analyzed for various purposes such as clinical trial supplementation, product targeting, disease prediction algorithm learning, etc., which provides an analysis base that is more realistic and immediate than existing manual-centered clinical data.

As described above, the present disclosure is applicable to various fields including public welfare, private consumption, data economy, insurance and pharmaceutical industries, and can be established as a convergence-type welfare platform technology that possesses both social and economic values beyond a simple healthcare solution.

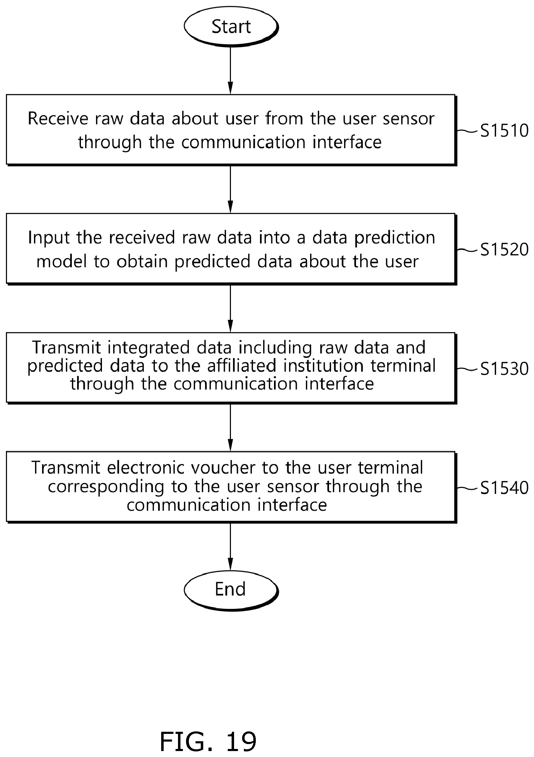

FIG. 19 is a flowchart illustrating an operation of the electronic device 100 according to an embodiment of the present disclosure.

Referring to FIG. 19, the processor may receive raw data RD about a user from the user sensor 10 through the communication interface (S1510).

The processor may input the received raw data RD into a data prediction model 121 to obtain predicted data (PD) about the user (S1520).

The processor may transmit the integrated data (ID), including raw data RD and predicted data (PD), to the affiliated institution terminal 30 through a communication interface (S1530).

The processor may transmit the electronic voucher EV to the user terminal 20 corresponding to the user sensor 10 through the communication interface (S1540).

According to the present disclosure, an embodiment may comply with the Social Welfare Services Act and welfare program standards, prevent confusion by differentiating from government programs under the names of "Green" and "AI", obtain explicit user consent (electronic signature) according to Article 15 of the Personal Information Protection Act, and disclose data processing purposes in advance. In addition, it may comply with Article 7 of the Act on Promotion of the Provision and Use of Public Data, specify a re-identification prohibition contract and a limitation on the purpose of data use, comply with Article 9 of the Act on Collection and Use of Donations, manage donations as a registered corporation, comply with Article 50 of the Act on Promotion of Information and Communications Network Utilization and Information Protection, etc., and specify an opt-out option. In addition, it may specify that it is "not medical diagnosis" and insert a warning message saying that "medical judgment should be made at a medical institution" so as not to violate the Medical Service Act (Monopoly on Medical treatment by Medical Professional).

Meanwhile, the various embodiments described above may be implemented by combining two or more embodiments without conflicting with each other.

Specifically, each of a plurality of operations, steps and configurations for implementing one embodiment may be implemented by another embodiment or an operation or step of another embodiment may follow the last operation or step of one embodiment, but is not limited thereto.

Meanwhile, the computer instructions or the computer program for performing the processing operations of various embodiments described above may be stored in a non-transitory computer-readable medium. When the computer instructions or the computer program stored in the non-transitory computer-readable medium are executed by a processor of a specific device, the specific device performs the processing operation according to various embodiments described above.

The non-transitory computer-readable medium refers to a medium that stores data semi-permanently and is readable by a device, rather than a medium that stores data only for a short time such as registers, caches, memories, etc. Specific examples of the non-transitory computer-readable medium may include CDs, DVDs, hard disks, Blu-ray discs, USBs, memory cards, ROMs, etc.

According to an embodiment, the method according to various embodiments disclosed in this document may be included and provided in a computer program product. The computer program product may be traded between a seller and a buyer as a commodity. The computer program product may be distributed in the form of a device-readable storage medium (e.g., compact disc read only memory (CD-ROM)), or may be distributed online (e.g., by downloading or uploading) through an application store (e.g., Play StoreTM) or directly between two user devices (e.g., smartphones). In the case of online distribution, at least part of the computer program product (e.g., downloadable app) may be temporarily generated or stored in a machine-readable storage medium such as a memory of a manufacturer's server, an application store server, or a relay server.

Although the preferred embodiments of the present disclosure have been illustrated and described above, the present disclosure is not limited to the above-described specific embodiments, and various modifications can be made by those skilled in the art to which the present disclosure pertains without departing from the scope of the present disclosure as claimed in the claims, and such modifications should not be understood separately from the technical spirit or prospect of the present disclosure.

## Claims

1. An electronic device for implementing an AI green welfare card system based on user data collection and provision comprising:
a communication interface for performing communication connection with an external device;
a memory for storing user data and a data prediction model; and
a processor connected to the communication interface and the memory to perform operations of collecting, processing, and providing user data,
wherein the processor
receives raw data about a user from a user sensor through the communication interface,
inputs the received raw data to the data prediction model to obtain predicted data about the user,
transmits integrated data including the raw data and the predicted data to an affiliated institution terminal through the communication interface, and
transmits an electronic voucher to a user terminal corresponding to the user sensor through the communication interface.

2. The electronic device of claim 1, wherein the processor receives raw data including at least one of an age, a gender, a location, a respiratory rate and a pulse rate of users from the user sensor through the communication interface.

3. The electronic device of any one of the proceeding claims, wherein the processor obtains predicted data on a body temperature and blood pressure of the user based on a value output by inputting the raw data into the data prediction model.

4. The electronic device of any one of the proceeding claims, wherein the processor replaces the user identifier corresponding to the integrated data with a predetermined identifier to obtain anonymized integrated data corresponding to the integrated data or limit essential data according to a principle of data minimization.

5. The electronic device of any one of the proceeding claims, wherein, when a target data request is received from the affiliated institution terminal through the communication interface, the processor identifies, among the integrated data, target data to be transmitted to the affiliated institution terminal based on the target data request, and
transmits the target data to the affiliated institution terminal that has requested the target data through the communication interface.

6. The electronic device of any one of the proceeding claims, wherein the processor transmits an electronic voucher corresponding to a high amount to the user terminal corresponding to the user sensor through the communication interface as the amount of raw data received from the user sensor through the communication interface increases, and
transmits an electronic voucher corresponding to a low amount to the user terminal corresponding to the user sensor through the communication interface as the amount of raw data received from the user sensor through the communication interface decreases.

7. The electronic device of any one of the proceeding claims, wherein the processor generates a customized health management plan for the user based on the raw data received from the user sensor through the communication interface, and
transmits the generated customized health management plan to the user terminal,
wherein the customized health management plan includes at least one health management plan of food intake, exercise, and sleep.

8. The electronic device of any one of the proceeding claims, wherein the processor performs outlier detection and quality score calculation for health data,
detects outliers in the health data using a Z-score or an IQR method, and
calculates a quality score based on accuracy, completeness, and consistency of the health data.

9. The electronic device of any one of the proceeding claims, wherein the processor periodically evaluates performance of an artificial intelligence model used for analyzing the health data, and
retrains the artificial intelligence model using new data when the performance of the artificial intelligence model falls below a preset criterion.

10. The electronic device of any one of the proceeding claims, wherein the processor receives raw data including heart rate and respiratory rate from a user sensor through the communication interface,
obtains predicted data on body temperature and blood pressure by inputting the raw data into the CRNN-based data prediction model,
generates integrated data by anonymizing the raw data and the predicted data using dynamic pseudonymization,
transmits the same to an affiliated institution terminal in accordance with an HL7 FHIR standard, and
issues an electronic voucher proportional to the amount of provided data based on blockchain technology to transmit the same to the user terminal.
